(19) Europäisches Patentamt
European Patent Office
Office européen des brevets

(11) Publication number: **0 211 598 B1**

(12) # EUROPEAN PATENT SPECIFICATION

(45) Date of publication of patent specification: **15.12.93**

(51) Int. Cl.5: **C07D 493/08**, A01N 43/90, A61K 31/335, C07C 31/22, C07C 35/02, C07C 43/164, C07F 7/08, //(C07D493/08, 319:00,319:00)

(21) Application number: **86305820.2**

(22) Date of filing: **29.07.86**

(54) **Pesticidal compounds.**

(30) Priority: **30.07.85 GB 8519212**
**12.09.85 GB 8522601**
**12.03.86 GB 8606130**

(43) Date of publication of application:
**25.02.87 Bulletin 87/09**

(45) Publication of the grant of the patent:
**15.12.93 Bulletin 93/50**

(84) Designated Contracting States:
**AT BE CH DE FR GB IT LI LU NL SE**

(56) References cited:
**US-A- 3 686 224**
**ZA-A- 8 500 734**

**EUROPEAN JOURNAL OF MEDICINAL CHEMISTRY-CHIMICA THERAPEUTICA, vol. 13, no. 3, May-June 1978, pages 207-212, Paris, FR; G.H. COOPER et al.: "Structure-activity relations in 2,6,7-trioxa-1-phosphabicyclo (2,2,2) octanes and related compounds"**

(73) Proprietor: **THE WELLCOME FOUNDATION LIMITED**
**Unicorn House**
**160 Euston Road**
**London NW1 2BP(GB)**

Proprietor: **THE REGENTS OF THE UNIVERSITY OF CALIFORNIA**
**2199 Addison Street**
**Berkeley, California 94720(US)**

(72) Inventor: **Casida, John Edward**
**1570 La Vereda Road**
**Berkeley**
**California 94708(US)**
Inventor: **Palmer, Christopher John**
**37 Clapgate Lane**
**Ipswich Suffolk(GB)**
Inventor: **Larkin, John Patrick**
**Harrow Road**
**Leighton Buzzard Bedfordshire(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3. 10/3.6/3.3. 1)

AGRICULTURAL AND BIOLOGICAL CHEMISTRY, vol. 46, no. 2, 1982, pages 411-418, Tokyo, JP; Y. OZOE: "Synthesis and some spectral characteristics of bicyclic phosphate, GABA antagonists"

J. Agric. Food Chem. 33, 976 (1985)

Inventor: Smith, Ian Harold
11 Moor End Close
Eaton Bray Bedfordshire(GB)

(74) Representative: Goldin, Douglas Michael et al
J.A. KEMP & CO.
14, South Square
Gray's Inn
London WC1R 5EU (GB)

## Description

The present invention relates to novel chemical compounds having pesticidal activity, to methods for their preparation, to compositions containing them and to their use in the control of pests. More particularly the invention relates to a class of 1,3,4-tri-substituted-2,6,7-trioxabicyclo[2,2,2]octanes.

The use of certain 2,6,7-trioxabicyclo[2,2,2]octanes as pesticides is disclosed in European Patent Application No. 152 229; Similar compounds are also disclosed in J. Agric. Food Chem. (1985) 33, 976-980, and ZA-A-85/00734. Other 2,6,7-trioxabicyclo[2.2.2]octanes for use as herbicides are disclosed in US-A-3,686,224. It has now been discovered that derivatives of these compounds having substituents at the 3-position have interesting pesticidal activity.

Accordingly the present invention provides a compound of the formula (I):

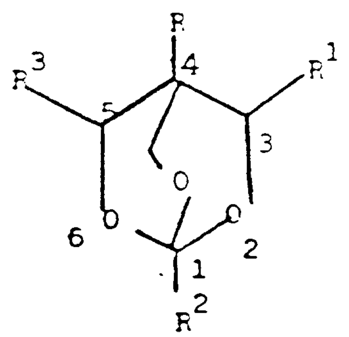

(I)

wherein R is $C_{2-10}$ alkyl, alkenyl or alkynyl, each optionally substituted by, or methyl substituted by, cyano, $C_{3-4}$ cycloalkyl,halo, $C_{1-4}$ alkoxy or a group S(O)m $R^4$ where $R^4$ is $C_{1-4}$ alkyl and m is 0, 1 or 2, or Ris $C_{3-10}$ cycloalkyl, $C_{4-10}$ cycloalkenyl or phenyl, each optionally substituted by $C_{1-4}$ alkoxy, $C_{1-3}$ alkyl, $C_{2-4}$ alkynyl,halo, cyano or a group S(O)m $R^4$ as defined hereinbefore; $R^1$ is halo, $C_{1-3}$ alkyl, $C_{2-3}$ alkenyl or alkynyl each optionally substituted by halo, cyano, $C_{1-4}$ alkoxy, alkyl carbalkoxy containing up to 6 carbon atoms, a group S(O)m $R^4$ as defined hereinbefore or alkynyl substituted by tri-$C_{1-4}$ alkylsilyl, or $R^1$ is cyano, spiro-cyclopropyl, gem dimethyl, gem dicyano, gem diethynyl, oxo or methylene optionally substituted by cyano or $C_{1-3}$ alkyl optionally substituted by fluorine, or $R^1$ and R and the carbon atoms to which they are attached form a $C_{5-7}$ carbocyclic ring optionally substituted by halo, $C_{1-3}$ alkyl or alkoxy or $C_{2-3}$ alkenyl, $R^2$ is phenyl, $C_{5-10}$ cycloalkyl or cycloalkenyl each optionally substituted by halo, cyano, azido, tetrazolyl, a group $SO_2R^5$ wherein $R^5$ is amino or di-$C_{1-4}$ alkylamino, a group $COR^6$ wherein $R^6$ is $C_{1-4}$ alkoxy, benzyloxy, amino or di-$C_{1-4}$ alkylamino, nitro, $C_{1-3}$ alkyl optionally substituted by halo, cyano or ethynl, or $C_{2-3}$ alkenyl or ethynyl each optionally substituted by halo or trl-$C_{1-4}$ alkylsilyl, and $R^3$ is hydrogen, $C_{1-3}$ alkyl, $C_{2-3}$ alkenyl or alkynyl each optionally substituted by cyano, $C_{1-4}$ alkylthio, $C_{1-4}$ alkoxy or halo, or $R^3$ is cyano or halo.

Suitably R is propyl, butyl, pentyl, $C_{2-5}$ alkenyl or alkynyl, $C_{5-7}$ cycloalkyl or phenyl each optionally substituted by one to three fluoro, chloro or bromo. Most suitably R is n-propyl, n-butyl, i-butyl, s-butyl, t-butyl, cyclopentyl or cyclohexyl and preferably R is n-propyl, n-butyl, i-butyl, t-butyl or cyclohexyl.

Suitably $R^1$ is cyano, ethynyl or methyl or ethyl optionally substituted by cyano, methoxy, methylthio or fluoro. Most suitably $R^1$ is methyl, cyano, ethynyl, trifluoromethyl or ethyl. Preferably $R^1$ is methyl, trifluoromethyl, cyano or ethynyl.

When $R^2$ is phenyl, $C_{5-10}$ cycloalkyl or cycloalkenyl substituted by an acidic or basic group, salts can be formed of compounds of the formula (I). The present invention includes salts of the compounds of formula (I). The preparation of such salts is carried out by methods well known to those skilled in the art. Typical salts include acid addition salts, such as those from mineral acids, and basic salts, such as those from the alkali earth metals.

Suitably $R^2$ is cyclohexyl, cycloheptyl, cyclooctyl or phenyl optionally substituted at the 3-,4- and/or 5-position by halo, cyano, $C_{2-3}$ alkynyl, azido or nitro and/or at the 2- and/or 6-position by fluoro. Most suitably $R^2$ is cyclohexyl or phenyl optionally substituted at the 3-,4- and/or 5-position by chloro, bromo, iodo, cyano or ethynyl. Preferably $R^2$ is phenyl substituted at the 4-position by chlorine, bromine, ethynyl or cyano.

Suitably $R^3$ is hydrogen or methyl.

Preferably $R^3$ is hydrogen

Suitable compounds of the formula (I) include those of the formula (IA)

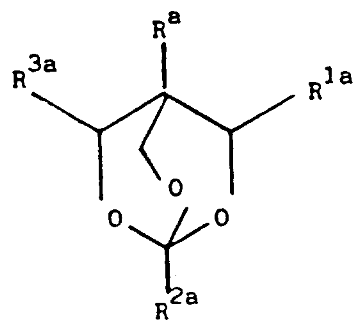

(IA):

wherein $R^a$ is $C_{2-4}$ alkyl, alkenyl or alkynyl, $C_{5-10}$ cycloalkyl or phenyl, each optionally substituted by cyano or $C_{1-4}$ alkoxy, $R^{1a}$ is cyano or $C_{1-3}$ alkyl, $C_{2-3}$ alkenyl or alkynyl each optionally substituted by cyano, $C_{1-4}$ alkoxy, $C_{1-4}$ alkylthio or halo, or $R^{1a}$ is cyano, gem dimethyl or $R^{1a}$ and $R^a$ and the carbon atoms to which they are attached form a $C_{5-7}$ carbocyclic ring optionally substituted by $C_{1-3}$ alkyl or alkoxy; $R^{2a}$ is phenyl, $C_{5-10}$ cycloalkyl or cycloalkenyl each optionally substituted and $R^{3a}$ is hydrogen, $C_{1-3}$ alkyl, $C_{2-3}$ alkenyl or alkynyl each optionally substituted by cyano, $C_{1-4}$ alkylthio, $C_{1-4}$ alkoxy or halo.

Suitably $R^a$ is propyl, butyl, $C_{5-7}$ cycloalkyl or phenyl. Most suitably $R^a$ is n-propyl, butyl, cyclopentyl or cyclohexyl and preferably $R^a$ is propyl, butyl or cyclohexyl.

Suitably $R^{1a}$ is cyano, methyl or ethyl optionally substituted by cyano, methoxy, methylthio or fluoro. Preferably $R^{1a}$ is methyl or ethyl.

Suitable substituents for $R^{2a}$ include halo, cyano, azido, nitro, $C_{1-3}$ alkyl optionally substituted by halo or $C_{2-3}$ alkenyl or alkynyl each optionally substituted by halo.

Suitably $R^{2a}$ is cyclohexyl, cycloheptyl or phenyl optionally substituted at the 3-or 4-position by halo, cyano, azido or nitro. Most suitably $R^{2a}$ is cyclohexyl or phenyl optionally substituted at the 4-position by chlorine, bromine or cyano.

Preferred compounds of the present invention include
1-(4-bromophenyl)-4-cyclohexyl-3-methyl-2,6,7-trioxabicyclo[2,2,2]octane,
1-(4-chlorophenyl)-3-methyl-4-iso-propyl-2,6,7-trioxabicyclo[2,2,2]octane,
1-(4-bromophenyl)-3-methyl-4-n-propyl-2,6,7-trioxabicyclo[2,2,2]octane,
1-(4-chlorophenyl)-3-methyl-4-n-propyl-2,6,7-trioxabicyclo[2,2,2]octane,
1-cyclohexyl-3-methyl-4-iso-propyl-2,6,7-trioxabicyclo[2,2,2]octane,
1-(4-bromophenyl)-4-t-butyl-3-methyl-2,6,7-trioxabicyclo[2,2,2]octane,
1,4-dicyclohexyl-3-methyl-2,6,7-trioxabicyclo[2,2,2]octane,
4-t-butyl-1-(4-chlorophenyl)-3-methyl-2,6,7-trioxabicyclo[2,2,2] octane.
1-(4-ethynylphenyl)-3-methyl-4-n-propyl-2,6,7-trioxabicyclo[2,2,2]octane
3-methyl-4-n-propyl-1-[4-(2-trimethylsilylethynyl)-phenyl]-2,6,7-trioxabicyclo [2,2,2]octane
1-(4-ethynylphenyl)-4-n-propyl-3-trifluoromethyl-2,6,7-trioxabicyclo[2,2,2]octane
4-n-propl-3-trifluoromethyl-1-[4-(2-trimethylsilylethynyl)-phenyl]-2,6,7-trioxabicyclo[2,2,2]octane
1-(4-chlorophenyl)-4-n-propyl-3-trifluoromethyl-2,6,7-trioxabicyclo[2,2,2]octane
1-(4-bromophenyl)-4-n-propyl-3-trifluoromethyl-2,6,7-trioxabicyclo[2,2,2]octane
1-(4-chlorophenyl)-4-cyclohexyl-3-trifluoromethyl-2,6,7-trioxabicyclo[2,2,2]octane
1-(4-bromophenyl)-4-cyclohexyl-3-trifluoromethyl-2,6,7-trioxabicyclo[2,2,2]octane
1-(4-bromophenyl)-3-ethenyl-4-n-propyl-2,6,7-trioxabicyclo[2,2,2]octane
1-cyclohexyl-4-n-propyl-3-trifluoromethyl-2,6,7-trioxabicyclo[2,2,2]octane
1-(4-bromophenyl)-3-cyano-4-n-propyl-2,6,7-trioxabicyclo[2,2,2]octane
3-cyano-1-(4-ethynylphenyl)-4-n-propyl-2,6,7-trioxabicyclo[2,2,2]octane
1-(4-bromophenyl)-4-cyclohexyl-3-methoxymethyl-2,6,7-trioxabicyclo[2,2,2]octane
1-cyclohexyl-3-methyl-4-n-propyl-2,6,7-trioxabicyclo[2,2,2]octane
1-(4-bromophenyl)-4-t-butyl-3-ethyl-2,6,7-trioxabicyclo[2,2,2]octane
4-t-butyl-1-cyclohexyl-3-methyl-2,6,7-trioxabicyclo[2,2,2]octane
1-(4-chlorophenyl)-3,5-dimethyl-4-n-propyl-2,6,7-trioxabicyclo[2,2,2]octane
1-(4-bromophenyl)-3,5-dimethyl-4-n-propyl-2,6,7-trioxabicyclo[2,2,2]octane
1-cyclohexyl-3,5-dimethyl-4-n-propyl-2,6,7-trioxabicyclo[2,2,2]octane
1-(4-cyanophenyl-4-n-propyl-3-trifluoromethyl-2,6,7-trioxabicyclo[2,2,2]octane
3-cyano-1-(4-cyanophenyl)-4-isopropyl-2,6,7-trioxabicyclo[2,2,2]octane
4-t-butyl-3-cyano-1-(4-iodophenyl)-2,6,7-trioxabicyclo[2,2,2]octane

4-t-butyl-3-cyano-1-[4-(2-trimethylsilylethynyl)-phenyl]-2,6,7-trioxabicyclo[2,2,2]octane
1-(4-bromophenyl)-4-n-butyl-3-methyl-2,6,7-trioxabicyclo[2,2,2]octane
4-n-butyl-1-(4-chlorophenyl)-3-methyl-2,6,7-trioxabicyclo[2,2,2]octane
4-t-butyl-3-cyano-1-(4-ethynylphenyl)-2,6,7-trioxabicyclo[2,2,2]octane
1-(4-cyanophenyl)-3-ethenyl -4-isopropyl-2,6,7-trioxabicyclo[2,2,2]octane
1-(4-bromo-3,5-dichlorophenyl)-4-n-propyl-3-trifluoromethyl-2,6,7-trioxabicyclo[2,2,2]octane,
1-(4-bromo-3-chlorophenyl)-4-n-propyl-3-trifluoromethyl-2,6,7-trioxabicyclo [2,2,2)octane
Particularly preferred compounds include
1-(4-bromophenyl)-3-methyl-4-n-propyl-2,6,7-trioxabicyclo[2,2,2]octane
1-(4-bromophenyl)-4-cyclohexyl-3-methyl-2,6,7-trioxabicyclo[2,2,2]octane
1-(4-chlorophenyl)-3-methyl-4-n-propyl-2,6,7-trioxabicyclo[2,2,2]octane
1-(4-bromophenyl)-4-t-butyl-3-methyl-2,6,7-trioxabicyclo[2,2,2]octane
1-(4-chlorophenyl)-4-t-butyl-3-methyl-2,6,7-trioxabicyclo[2,2,2]octane
1-(4-bromophenyl)-4-n-propyl-3-trifluoromethyl-2,6,7-trioxabicyclo[2,2,2]octane
1-(4-ethynylphenyl)-4-n-propyl-3-trifluoromethyl-2,6,7-trioxabicyclo[2,2,2]octane
1-(4-ethynylphenyl)-3-methyl-4-n-propyl-2,6,7-trioxabicyclo[2,2,2]octane
1-(4-cyanophenyl)-4-n-propyl-3-trifluoromethyl-2,6,7-trioxabicyclo[2,2,2]octane
1-(4-bromophenyl)-4-n-butyl-3-methyl-2,6,7-trioxabicyclo[2,2,2]octane
4-n-butyl-1-(4-chlorophenyl)-3-methyl-2,6,7-trioxabicyclo[2,2,2]octane
4-t-butyl-3-cyano-1-(4-ethynylphenyl)-2,6,7-trioxabicyclo[2,2,2]octane
1-(4-cyanophenyl)-3-ethenyl-4-isopropyl-2,6,7-trioxabicyclo[2,2,2]octane, or
4-n-Butyl-1-(4-bromophenyl)-3-trifluoromethyl-2,6,7-trioxabicyclo[2.2.2]octane,
4-n-Butyl-1-(4-indophenyl)-3-methyl-2,6,7-trioxabicyclo[2.2.2]octane,
4-n-Butyl--3-methyl-1-[4-(2-trimethylsilyl ethynyl)phenyl]-2,6,7-trioxabicyclo[2.2.2]octane,
4-n-Butyl-1-(4-ethynyl phenyl)-3-methyl-2,6,7-trioxabicyclo[2.2.2]octane,
4-t-Butyl-3-cyano-1-cyclohexyl-2,6,7-trioxabicyclo[2.2.2]octane,
1-(4-Bromophenyl)-3-cyano-4-n-propyl-2,6,7-trioxabicyclo[2.2.2]octane,
3-Cyano-1-(4-iodophenyl)-4-n-propyl-2,6,7-trioxabicyclo[2.2.2]octane,
3-Cyano-4-n-propyl-1-[4-(2-trimethylsilylethynyl)phenyl]-2,6,7-trioxabicyclo[2.2.2]octane, or
3-Cyano-1-(4-ethynylphenyl)-4-n-propyl-2,6,7-trioxabicyclo-[2.2.2]octane.

In a further aspect, the present invention provides a process for the preparation of a compound of the formula (I). The process for the preparation of a compound of the formula (I) may be any method known in the art for preparing analogous compounds, for example by the condensation of a triol of the formula (II) with an orthocarboxylate of the formula $R^2C(OR^7)_3$.

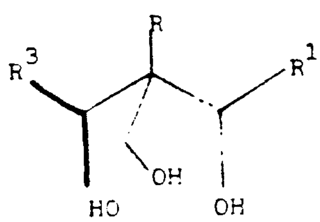

(II)

wherein R to $R^3$ are as hereinbefore defined and $R^7$ is $C_{1-4}$ alkyl, phenyl or $C_{7-8}$ aralkyl. Suitably $R^7$ is methyl or ethyl, preferably methyl. The reaction is normally carried out in the presence of an acid such as a mineral acid conveniently hydrochloric acid, or a sulphonic acid derivative, such as toluene sulphonic acid,or an acid resin, or in the presence of a trialkyl amine, such as triethylamine, at an elevated temperature, for example between 50 and 200 °C, conveniently between 120 and 170 °C. The reaction may conveniently be carried out in the absence of a solvent but a suitable solvent may be added if desired.

The triol of the formula (II) may be prepared:

(i) from the corresponding triol where $R^1$ and/or $R^3$ are hydrogen via a protected aldehyde which is reacted with a reagent, such as a Grignard reagent, which is suitable for lengthening the carbon chain followed by deprotection, i.e. as represented in Scheme 1.

In certain cases, it may be convenient to prepare triol derivatives where $R^1$, $R^3$ are hydrogen and one of the hydroxy groups is protected, by reduction of an ester of the formula (III):

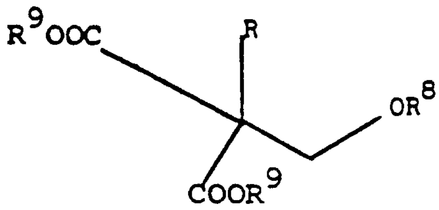

(III)

wherein $R^8$ is a protecting group such as benzyl and $R^9$ is $C_{1-4}$ alkyl. This reduction is suitably carried out by a complex hydride such as lithium aluminium hydride in an inert solvent conveniently an ether. The compound of the formula (III) may be prepared from the corresponding compound RCH $(CO_2R^9)_2$ by reaction with a compound $XCH_2OR^8$, wherein X is a leaving group such as a halogen, in the presence of a strong base, such as sodium hydride.

(ii) when it is required to prepare a compound of the formula (I) wherein $R^3$ is hydrogen, by the reduction of a compound of the formula (IV):

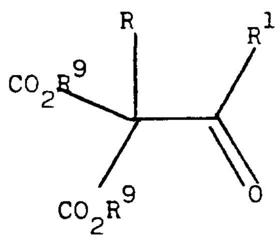

(IV)

wherein R, $R^1$ and $R^9$ are as hereinbefore defined. This reduction is suitably carried out by means of a complex hydride, such as lithium aluminium hydride in an inert solvent such as an ether, for example diethyl ether.

When R and $R^1$ are linked to form a carbocyclic ring, the compound of the formula (IV) is conveniently prepared by the reaction of a compound of the formula (V)

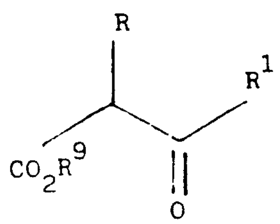

(V)

with a compound hal $CO_2R^9$, wherein R, $R^1$, and $R^9$ are as hereinbefore defined and hal is halogen, for example chlorine. This reaction is conveniently carried out in the presence of a Grignard reagent for example ethyl magnesium bromide, in an inert solvent such as an ether, for example tetrahydrofuran. Other compounds of the formula (IV) are conveniently prepared by the reaction of a compound RCH $(CO_2R^9)_2$ with a compound hal CO.$R^1$ wherein R, $R^1$, $R^9$ and hal are as hereinbefore defined or a trifluoroacylating agent such as trifluoroacetic anhydridetrifluoroacetic acid or ethyl trifluoroacetate. This reaction is conveniently carried out in the presence of a strong base, such as a metal hydride in a non-polar solvent, for example an aromatic hydrocarbon such as benzene or toluene.

The compounds of the formula (I) may also be prepared by the cyclisation of a compound of the formula (VI)

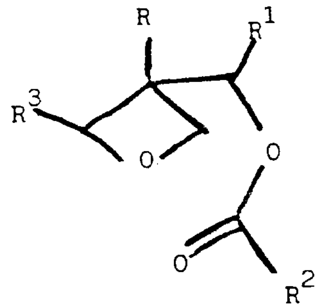

(VI)

wherein R to $R^3$ are as hereinbefore defined in the presence of an acid catalyst.Boron trifluoride etherate is a particularly preferred acid catalyst for this cyclisation which will normally be carried out in an inert solvent, such as a halogenated hydrocarbon, conveniently dichloromethane, at below ambient temperature, for example between -100 and 0 ° C and conveniently between -70 and-50 ° C.

The compounds of the formula (VI) may be prepared by the reaction of compounds of the formula (VII) and (VIII):

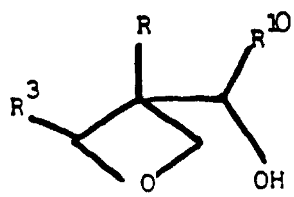

(VII)

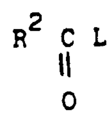

where $R^{10}$ is a group $R^1$ and R to $R^3$ are as hereinbefore defined and L is leaving group such as halo. This reaction conveniently takes place in an inert solvent in the presence of base at a non-extreme temperature. Halogenated hydrocarbons, such as dichloromethane are particularly suitable solvents, pyridine is a preferred base and the reaction will conveniently be carried out at between -50 and 100 ° C, preferably at 0 ° C.

The compounds of the formula (VII) may in turn be prepared from compounds of the formula (II) by reaction with diethyl carbonate in the presence of a strong base, for example potassium hydoxide, in a polar solvent, such as an alcohol, for example ethanol, at an elevated temperature, for example between 50 and 100 ° C. This is a preferred method of making compounds of the formula (VII) wherein $R^1 = R^{10} = CF_3$.

The compounds of the formula (VII) may alternatively be prepared by the reaction of a Grignard reagent $R^1$ Mg Hal or an $C_{2-4}$ alkynyl lithium compound or sodium oyanide with a compound of the formula (IX)

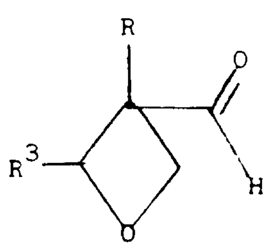

(IX)

wherein R,$R^1$ and $R^3$ are as hereinbefore defined and Hal is a halogen atom such as bromine or iodine. This reaction is conveniently carried out in an inert solvent, suitably an ether for example diethyl ether or dioxane at a non-extreme temperature, for example between -50 and 50 ° C and preferably between -10 ° and 10 ° C. The alkynyl lithium compound is conveniently present as a complex, for example with ethylenedianime. The sodium cyanide is conveniently added as an aqueous solution. The compounds of the formula (IX) may be prepared by oxidation of compounds of the formula (VII) wherein $R^{10}$ is hydrogen by using oxalyl chloride and dimethyl sulphoxide in an inert solvent, such as a halogenated hydrocarbon, for example dichloromethane, followed by a base such as triethylamine or by using pyridinium chlorochromate in an inert

solvent, such as a halogenated hydrocarbon, for example dichloromethane.

The compounds of the formula (VII) wherein $R^{10}$ is hydrogen may be prepared in an analogous manner from compounds of formula (II) to the preparation of the compounds of the formula (VII) where $R^{10}$ is trifluoromethyl.

One compound of the formula (I) may be converted to another com pound of the formula (I) by methods well known to those skilled in the art. Thus those compounds of the formula (I) wherein $R^2$ is an ethynylphenyl group may be prepared from the corresponding iodophenyl group via the trimethylsilylethynylphenyl intermediate.

The compounds of Formula (I) may be used to control arthropods such as insect and acarine pests. Thus in a further aspect, the, present invention provides a method of controlling arthropods on animals which comprises the administration of an arthopodially effective amount of a compound of the formula (I) to the animal.

The compounds of Formula (I) may be used for such purposes by application of the compounds themselves or in diluted form in known fashion as a dip, spray, lacquer, foam, dust, powder, aqueous suspension, paste, gel, shampoo, grease, combustible solid, vapourising emanator such as a vapourising mat, wettable powder, granule, aerosol, emulsifiable concentrate, oil suspensions, oil solutions, impregnated article or pour on formulation. Dip concentrates are not applied per se, but diluted with water and the animals immersed in a dipping bath containing the dip wash. Sprays may be applied by hand or by means of a spray race or arch or by means of space spraying machinery. The animal; plant or surface being treated may be saturated with the spray by means of high volume application or superficially coated with the spray by means of light or ultra low volume application. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material against which animals rub and transfer the material to their skins. Pour-on formulations are dispensed as a unit of liquid of small volume on to the backs of animals such that all or most of the liquid is retained on the animals.

The compounds of Formula (I) may be formulated either as formulations ready for use on the animals, plants or surface, as space sprays or aerosols, or as formulations requiring dilution prior to application, but both types of formulation comprise a compound of Formula (I) in intimate admixture with one or more carriers or diluents. The carriers may be liquid, solid or gaseous or comprise mixtures of such substances, and the compound of Formula (I) may be present in a concentration of from 0.025 to 99% w/v depending upon whether the formulation requires further dilution.

Dusts, powder and granules comprise the compound of Formula (I) in intimate admixture with a powdered solid inert carrier for example suitable clays, kaolin, silicone dioxide, talc, mica, chalk, gypsum, vegetable carriers, starch and diatomaceous earths.

Sprays of a compound of Formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 1 to 90% w/v of the composition and may be selected from kerosene, ketones, alcohols, xylene, aromatic naphtha, aromatic and aliphatic esters and other solvents known in the formulating art. The concentration of emulsifiers may be varied within wide limits but is preferably in the range of 0.5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates.

Wettable powders comprise an inert solid carrier, one or more surface active agents, and optionally stabilisers and/or anti-oxidants.

Wettable powders and emulsifiable concentrates will normally contain from 1 to 95% by weight of the active ingredient, and are diluted, for example with water, before use.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of Formula (I) in intimate admixture with a dispersing agent and one or more surface active agents.

Aqueous suspensions of a compound of Formula (I) may comprise a suspension in water together with suspending, stabilizing or other agents. The suspensions or solutions may be applied per se or in a diluted

form in known fashion.

Greases (or ointments) may be prepared from vegetable oils, synthetic esters of fatty acids or wool fat together with an inert base such as soft paraffin. A compound of Formula (I) is preferably distributed uniformly through the mixture in solution or suspension. Greases may also be made from emulsifiable concentrates by diluting them with an ointment base.

Pastes and shampoos are also semi-solid preparations in which a compound of Formula (I) may be present as an uniform dispersion in a suitable base such as soft or liquid paraffin or made on a non-greasy basis with glycerin, mucilage or a suitable soap. As greases, shampoos and pastes are usually applied without further dilution they should contain the appropriate percentage of the compound of Formula (I) required for treatment.

Aerosol sprays may be prepared as a simple solution of the active ingredient in the aerosol propellant and cosolvent such as halogenated alkanes and the solvents referred to above, respectively. Pour-on formulations may be made as a solution or suspension of a compound of Formula (I) in a liquid medium. An avian or mammal host may also be protected against infestation of acarine ectoparasites by means of carrying a suitably-moulded, shaped plastics article impregnated with a compound of Formula (I). Such articles include impregnated collars, tags, bands, sheets and strips suitably attached to appropriate parts of the body.

The concentration of the compound of Formula (I) to be applied to an animal will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited on an animal will vary according to the method of application, size of the animal, concentration of the compound in the applied formulation, factor by which the formulation is diluted and the nature of the formulation but in general will lie in the range of from 0.0001% to 0.5% except for undiluted formulations such as pour-on formulations which in general will be deposited at a concentration in the range from 0.1 to 20.0% and preferably 0.1 to 10%.

The compounds of formula (I) are also of use in the protection and treatment of plant species, in which case an effective insecticidal or acaricidal amount of the active ingredient is applied. The application rate will vary according to the compound chosen, the nature of the formulation, the mode of application, the plant species, the planting density and likely infestation and other like factors but in general, a suitable use rate for agricultural crops is in the range 0.001 to 3Kg/Ha and preferably between 0.01 and 1Kg/Ha. Typical formulations for agricultural use contain between 0.0001% and 50% of a compound of formula (I) and conveniently betwee 0.1 and 15% by weight of a compound of the formula (I).

Particular crops include cotton, wheat, maize, rice, sorghum, soya, vines, tomatoes, potatoes, fruit trees and spruce.

Dusts, greases, pastes, surface and space sprays and aerosol formulations are usually applied in a random fashion as described above and concentrations of 0.001 to 20% w/v of a compound of Formula (I) in the applied formulation may be used.

The compounds of formula (I) have been found to have activity against the common housefly (Musca domestica). In addition, compounds of formula (I) have activity against other arthropod pests including Tetranychus urticae Plutella xylostella, Culex spp. and Blattella germanica) The compounds of formula (I) are thus useful in the control of arthropods e.g. insects and acarines in any environment where these constitute pests, e.g. in agriculture, in animal husbandry, in public health and in domestic situations.

Insect pests include members of the orders Coleoptera (e.g. Anobium, Tribolium, Sitophilus, Diabrotica, Anthonomus or Anthrenus spp.), Lepidoptera (e.g. Ephestia, Plutella, Chilo, Heliothis, Spodoptera or Tineola spp.), Diptera (e.g. Musca, Aedes, Culex, Glossina, Stomoxys, Haematobia, Tabanos, Hydrotaea, Lucilia, Chrysomia, Callitroga, Dermatobia, Hypoderma, Liriomyza and Melophagus spp.), Phthiraptera (Malophaga e.g. Damalina spp. and Anoplura e.g. Pediculus humanus capitis, Pediculus humanus humanus, Phythirus pubis Linognathus and Haematopinus spp.), Hemiptera (e.g. Aphis, Bemisia, Aleurodes, Nilopavata, Nephrotetix or Cimex spp.), Orthoptera (e.g. Schistocerca or Acheta spp.), Dictyoptera (e.g. Blattella, Periplaneta or Blatta spp.), Hymenoptera (e.g. Solenopsis or Monomorium spp.), Isoptera (e.g. Reticulitermes spp.), Siphonaptera (e.g. Ctenocephalides or Pulex spp.), Thysanura (e.g. Lepisma spp.), Dermaptera (e.g. Forficula spp.) and Pscoptera (e.g. Peripsocus spp.).

Acarine pests include ticks, e.g. members of the genera Boophilus, Rhipicephalus, Amblyomma, Hyalomma, Ixodes, Haemaphysalis, Dermocentor and Anocentor, and mites and manges such as Sarcoptes scabiei, and Tetranychus, Psoroptes, Notoedres, Psorergates, Chorioptes and Demodex spp.

Compounds of the invention may be combined with one or more other active ingredients (for example pyrethroids, carbamates and organophosphates) and/or with attractants and/or with fungicides and the like. Furthermore, it has been found that the activity of the compounds of the invention may be enhanced by the

addition of a synergist or potentiator, for example: one of the oxidase inhibitor class of synergists, such as piperonyl butoxide or NIA 16388; a second compound of the invention; or a pyrethroid pesticidal compound. When an oxidase inhibitor synergist is present in a formula of the invention, the ratio of synergist to compound of Formula (I) will be in the range 25:1-1:25 eg about 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin).

It will be understood that what we will claim may comprise:

(a) compounds of Formula (I);

(b) processes for the preparation of compounds of Formula (I);

(c) insecticidal and acaricidal compositions comprising a compound of Formula (I) in admixture with a carrier;

(d) processes for the preparation of such pesticidal compositions;

(e) methods for the control of arthropod pests, such as insect or acarine pests comprising the application to the pest or its environment of a compound of Formula (I);

(f) synergised pesticidal compositions comprising a compound of Formula (I); and

(g) potentiating or non-potentiating mixtures of a compound of Formula (I) and another pesticidal compound;

(h) novel intermediates of the preparation of compounds of Formula (I).

The following Examples illustrate preferred aspects of the invention.

The physical data for each of the compounds of the formula (I) is provided in tables after the examples. All indicated temperatures are in ° Celsius.

## Example A:

1-Cyclohexyl-4-ethyl-3-methyl-2,6,7-trioxabicyclo[2,2,2]octane.

(i) Sodium hydride (24g. 50% dispersion in oil) was added to a stirred solution of diethyl ethylmalonate (94g.) in dry benzene (300ml.) at room temperature. The mixture was maintained at 60°C, with stirring, for one hour. The mixture was cooled and acetyl chloride (36ml.) was added and the reaction mixture was stirred at room temperature for three hours. The reaction mixture was poured into ice and the aqueous mixture was extracted with ether. The ether extracts were washed with water, dried over magnesium sulphate and the solvent was removed in vacuo.

Distillation gave diethyl 2-acetyl-2-ethylmalonate (54g.), a colourless oil (b.pt. 94° 1.5m.m.)

Nuclear magnetic resonance spectrum (NMR) was as follows: 'H(ppm from TMS in $CDC1_3$, integral, number of peaks, $J_{Hz}$): 4.25, 4H, q, 6; 2.36, 3H, s; 2.2, 2H, m; 1.4,6H,t,6; 1.0, 3H,t, 6

(ii) Lithium aluminium hydride (8.0g.) in dry diethyl ether (200ml) was stirred at 0°, under a current of dry nitrogen. Diethyl 2-acetyl-2-ethylmalonate (30g.) in dry ether (50ml) was added and the mixture was stirred at room temperature for three hours. The mixture was then refluxed, with stirring, for eight hours. A solution of sodium hydroxide (20g.) and potassium hydrogen phosphate (20g.) in water (150ml) was added carefully to the cooled reaction mixture. The pH was adjusted to 5.0 with glacial acetic acid. The solid was removed by filtration and washed with water (20 ml). The combined filtrates and washings were evaporated in vacuo. The residue was washed with acetone (3x100ml). The acetone washings were evaporated in vacuo. The residue was washed with chloroform (3x100ml) and the washings were evaporated in vacuo. 2-Ethyl-2-hydroxymethyl-butan-1,3-diol was obtained as a pale yellow oil (8.0g.) Nuclear magnetic resonance spectrum (NMR) was as follows: 'H (ppm from TMS in $CDC1_3$, integral, number of peaks, $J_{Hz}$): 4.2, 3H, broad singlet; 4.1, 1H, m; 3.8, 4H, m; 1.4, 5H, m; 1.0, 3H, m.

(iii) Trimethyl orthocyclohexylcarboxylate (1.1g) was added to 2-ethyl-2-hydroxymethyl-butan-1,3-diol (0.75g.) One drop of concentrated hydrochloric acid was added and the mixture was maintained at 130° for one hour, under a current of nitrogen. The volatile components were removed in vacuo (3.0mm.) at 130°.

The residue was purified by chromatography on alumina (Alumina Woelm TSC), eluting with 1:4 dichloromethane: hexane, saturated with ammonia. 1-Cyclohexyl-4-ethyl-3-methyl-2,6,7-trioxabicyclo-[2,2,2]octane was obtained as a colourless oil (0.3g.).

Gas-liquid chromatography (g.l.c.): OV210 at 150° produced one peak.

In an analogous manner, but where appropriate, starting with diethyl n-propylmalonate, diethyl i-propylmalonate or diethyl n-butylmalonate and acetyl chloride, propionyl chloride, butyryl chloride or methoxyacetyl chloride and carrying out the final condensation with trimethyl orthocyclohexylcarboxylate,

trimethyl 4-chloro-orthobenzoate or trimethyl 4-bromo-orthobenzoate, the following compounds were prepared:

1-(4-chlorophenyl)-4-ethyl-3-methyl-2,6,7-trioxabicyclo[2,2,2]octane
1-(4-bromophenyl)-4-ethyl-3-methyl-2,6,7-trioxabicylo[2,2,2]octane
1-(4-chlorophenyl)-4-n-propyl-3-methyl-2,6,7-trioxabicyclo[2,2,2]octane
1-cyclohexyl-4-n-propyl-3-methyl-2,6,7-trioxabicyclo[2,2,2]octane
1-cyclohexyl-4-i-propyl-3-methyl-2,6,7-trioxabicyclo[2,2,2]octane
1-(4-bromophenyl)-4-n-propyl-3-methyl-2,6,7-trioxabicyclo[2,2,2]octane
1-(4-chlorophenyl)-4-i-propyl-3-methyl-2,6,7-trioxabicylco[2,2,2]octane
1-(4-bromophenyl)-3-ethyl-4-n-propyl-2,6,7-trioxabicyclo[2,2,2]octane
1-cyclohexyl-3-ethyl-4-n-propyl-2,6,7-trioxabicyclo[2,2,2]octane
1-(4-bromophenyl)-3-ethyl-4-i-propyl-2,6.7-trioxabicyclo[2,2,2]octane
1-(4-bromophenyl)-3-n-propyl-4-i-propyl-2,6,7-trioxabicyclo[2,2,2]octane
1-cyclohexyl-3-n-propyl-4-i-propyl-2,6,7-trioxabicyclo[2,2,2]octane
1-(4-bromophenyl)-3-methoxymethyl-4-i-propyl-2,6,7-trioxabicyclo[2,2,2]octane
1-cyclohexyl-3-methoxymethyl-4-n-propyl-2,6,7-trioxabicyclo[2,2,2]octane
1-(4-bromophenyl)-3-methoxymethyl-4-n-propyl-2,6,7-trioxabicyclo[2,2,2]octane
1-cyclohexyl-3-methoxymethyl-4-i-propyl-2,6,7-trioxabicyclo[2,2,2]octan
1-(4-bromophenyl)-1-cyclohexyl-3-methoxymethyl-2,6,7-trioxabicyclo[2.2.2]octane.
1-(4-bromopheynl)-4-n-butyl-3-methyl-2,6,7-trioxabicyclo[2,2,2]octane
4-n-butyl-1-(4-chlorophenyl)-3-methyl-2,6,7-trioxabicyclo[2,2,2]octane.

Example B:

1-Cyclohexyl-3,4-diethyl-2,6,7-trioxabicyclo[2,2,2]octane.

(i) 2-Ethyl-2-hydroxymethyl-propan-1,3-diol (34g.), acetone (37ml.) and p-toluenesulphonic acid (0.5g.) were refluxed in benzene (140ml.) and water was removed using Dean and Stark apparatus. After ten hours refluxing the mixture was cooled and washed with saturated aqueous sodium hydrogen carbonate solution. The benzene solution was dried over anhydrous magnesium sulphate. The solvent was removed in vacuo. Distillation gave 2,2-dimethyl-5-ethyl-5-hydroxymethyl-1,3-dioxane (35g.), a colourless oil (bpt 80-2° 0.9mm)[2].

Nuclear magnetic resonance spectrum (NMR) was as follows: 'H (ppm from TMS in CDC1,, integral, number of peaks, $J_{Hz}$):

3.90, 2H, d, 6; 3.85, 4H, s; 3.10, 1H, t,6; 1.60, 6H, s; 1.55, 2H, m; 1.05, 3H, m.

(ii) 2,2-Dimethyl-5-ethyl-5-hydroxymethyl-1,3-dioxane (33g.) was added to a stirred suspension of pyridinium chlorochromate (122.6g.) and anhydrous sodium acetate (7.8g.) in dry dichloromethane (200ml.), at 0°, under a current of nitrogen. The mixture was stirred at room temperature for six hours. The mixture was diluted with dry ether (500ml) and the organic solution was decanted off. The oily residue was treated with ether and the combined extracts were evaporated in vacuo. The residue was purified by chromatography on silica, eluting with ether: hexane 1:3. 2,2-Dimethyl-5-ethyl-5-formyl-1,3-dioxane (30g.) was obtained as a colourless oil.

Gas-liquid chromatography (g.l.c.): OV210 at 130° produced one peak.

Nuclear magnetic resonance spectrum (NMR) was as follows: $^1$H (ppm from TMS in $CDC1_3$, integral, number of peaks, $J_{Hz}$) 9.6, 1H,s;

4.2, 2H,d,12; 3.8,2H,d,12; 1.4, 8H, m; 0.85, 3H, t, 6.

(iii) A solution of ethyl magnesium bromide (70ml, 1.3M in tetrahydrofuran) was stirred at 0°, under nitrogen. 2,2-Dimethyl-5-ethyl-5-formyl-1,3-dioxane (10.3g.) in dry tetrahydrofuran (20ml.) was added and the mixture was stirred at room temperature for three hours. The mixture was then refluxed, with stirring, for one hour. Saturated aqueous ammonium chloride solution was added to the cooled reaction mixture. The aqueous mixture was extracted with ether. The ether extracts were washed with water, dried over anhydrous magnesium sulphate and the solvent was removed in vacuo. The residue was purified by chromatography on silica gel, eluting with 40% ether hexane. 2,2-Dimethyl-5-ethyl-5-(1-hydroxy propyl)-1,3-dioxane was obtained as a colourless oil (10.0g).

Gas-liquid chromatography (g.l.c): OV210 at 140° produced one peak.

Nuclear magnetic resonance spectrum (NMR) was as follows: $^1$H (ppm from TMS in $CDC1_3$, integral, number of peaks, $J_{Hz}$):

3.70, 5H, m; 2.20, 1H, d, 6; 1.40, 10H m; 1.00, 6H, m.

(iv) 2,2-Dimethyl-5-ethyl-5-(1-hydroxypropyl)-1,3-dioxane (10g.) and Dowex 50x8-200 ion exchange resin ($H^+$ form) (1.0g.) in methanol (200ml) containing water (40ml) was refluxed with stirring for three hours. The mixture was filtered and the filtrate was evaporated in vacuo. 2-Ethyl-2-hydroxymethyl-pentan-1,3-diol (6.0g.) was obtained as a colourless viscous oil.

Nuclear magnetic resonance spectrum (NMR) was as follows: $^1H$ (p.p.m. from TMS in $CDC1_3$, integral, number of peaks, $J_{Hz}$):3.80, 5H, m; 3.00, 3H, m; 1.8-0.8,10H, m.

(v) From 2-ethyl-2-hydroxymethyl-pentan-1,3-diol(0.7g) using the method described in Example A, 1-cyclohexyl-3,4-diethyl-2,6,7-trioxabicyclol2,2,2]octane (0.3g.), a colourless oil, was obtained.

Gas-liquid chromatography (g.l.c.): OV210 at 180° produced one peak.

1-(4-chlorophenyl)-3,4-diethyl-2,6,7-trioxabicyclo[2,2,2]octane, was prepared in an analogous manner by substituting trimethyl 4-chloro-orthobenzoate for trimethyl orthocyclohexylcarboxylate in the final condensation.

1-cyclohexyl-4-t-butyl-3-methyl-2,6,7-trioxabicyclo[2,2,2]octane
1-(4-bromophenyl)-4-t-butyl-3-methyl-2,6,7-trioxabicyclo[2,2,2]octane
4-t-butyl-1-(4-chlorophenyl)-3-methyl-2,6,7-trioxabicyclo[2,2,2]octane and
1-(4-bromophenyl)-4-t-butyl-3-ethyl-2,6,7-trioxabicyclo[2,2,2]octane were
prepared by an analogous approach starting with 2-t-butyl-2-hydroxymethyl-propan-1,3-diol.
1-(4-bromophenyl)-3-ethenyl-4-n-propyl-2,6,7-trioxabicyclo(2,2,2)octane
was prepared in an analogous manner by substituting vinyl magnesium bromide for ethyl magnesium bromide in step (iii) and substituting triethylamine for hydrochloric acid in step (v)

Example C

1-(4-Bromophenyl)-4-cyclohexyl-3-methyl-2,6,7-trioxabicyclo[2,2,2]octane

(i) Diethyl cyclohexylmalonate (18.7g.) was added to a stirred suspension of sodium hydride (4.8g. 50% dispersion in oil) in dry tetrohydrofuran (50ml) under nitrogen. The mixture was refluxed, with stirring, for one hour. The mixture was cooled and benzyl chloromethyl ether (13.9g.) in dry tetrahydrofuran (50ml) was added and the mixture was refluxed, with stirring, for three hours. The mixture was cooled and poured into water. The aqueous mixture was extracted with ether. The ether extracts were washed with water, dried over anhydrous magnesium sulphate and evaporated in vacuo. Diethyl 2-benzyloxymethyl-2-cyclohexyl-malonate (30g.) was obtained as a brown oil and was used without further purification.

(ii) Diethyl 2-benzyloxymethyl-2-cyclohexyl-malonate (2g.) was added to a suspension of lithium aluminium hydride (0.63g.) in dry ether (30ml), at 0°, under nitrogen. The mixture was stirred at room temperature for twelve hours. Water (5 ml) was added carefully and the mixture was stirred for ten minutes. 10% sulphuric acid solution (10ml) was added and the mixture was extracted with ether. The ether extracts were washed with water, dried over anhydrous magnesium sulphate and evaporated in vacuo. The residue was purified by chromatography on silica, eluting with 1:1 ether: hexane. 2-Benzyloxymethyl-2-cyclohexyl-propan-1,3-diol was obtained as a colourless oil (1.0g.).

Gas-liquid chromatography (g.l.c.): OV210 at 230° produced one peak.

Nuclear magnetic resonance spectrum (NMR) was as follows: $^1H$ (ppm from TMS in $CDC1_3$, integral, number of peaks, $J_{Hz}$):

7.35, 5H, s; 4.55, 2H, s; 3.75, 4H, d, 6; 3.60, 2H, s; 2.90, 2H,t, 6; 2.00-0.90, 11H,m.

(iii) 2-Benzyloxymethyl-2-cyclohexyl-propan-1,3-diol(3g.),2,2-dimethoxypropane (8ml), p-toluenesulphonic acid (150mg.) and molecular sieves (type 4A) were heated in refluxing dry toluene (50ml.) for four hours. The mixture was cooled and filtered. The filtrate was diluted with ether and the ethereal solution was extracted with aqueous sodium hydrogen carbonate solution. The ethereal solution was washed with water, dried over anhydrous magnesium sulphate and evaporated in vacuo. The residue was purified by chromatography on silica, eluting with 1:5 ether:hexane. 5-Benzyloxymethyl-5-cyclohexyl-2,2-dimethyl-1,3-dioxane(3.0g) was obtained as a colourless oil.

Gas-liquid chromatography (g.l.c.):OV210 at 230° produced one peak.

Nuclear magnetic resonance spectrum (NMR) was as follows: $^1H$ (ppm from TMS in $CDC1_3$, integral, number of peaks);

7.30, 5H, s; 4.50, 2H, s; 3.70, 4H, s; 3.60, 2H,s; 2.00 - 0.9, 17H, m.

(iv) 5-Benzyloxymethyl-5-cyclohexyl-2,2-dimethyl-1,3-dioxane (3.0g.) in dry ether (10ml.) was added to liquid ammonia (100ml.) at -70°. Sodium (0.5g.) was added to the stirred solution. Stirring was maintained at -70°, for one hour. The mixture was then allowed to warm up to -30° and solid ammonium chloride (2.0g.) was added. The ammonia was removed from the reaction mixture under a current of

nitrogen. Water was added and the aqueous mixture was extracted with diethyl ether. The ethereal extracts were washed with water, dried over anhydrous magnesium sulphate and evaporated in vacuo. 5-Cyclohexyl-2,2-dimethyl-5-hydroxymethyl-1,3-dioxane was obtained as a cream solid (2.15g.) and was used without further purification.

Gas-liquid chromatography (g.l.c.): OV210 at 200° produced one peak.

Nuclear magnetic resonance spectrum (NMR) was as follows: $^1$H (p.p.m. from TMS in $CDC1_3$, integral, number of peaks):

3.80, 2H, s; 3.65, 4H,s; 2.30, 1H, s broad; 2.00 - 0.8, 17H, m.

(v) 5-Cyclohexyl-2,2-dimethyl-5-hydroxymethyl-1,3-dioxane (2.2g.) was added to a stirred suspension of pyridinium chlorochromate (6.1g.) and anhydrous sodium acetate (3.0g.) in dry dichloromethane (50ml.), at 0°, under a current of nitrogen. The mixture was stirred at room temperature for six hours. The mixture was diluted with dry ether (100ml) and the organic solution was decanted off. The oily residue was treated with ether and the combined extracts were evaporated in vacuo. 5-Cyclohexyl-2,2-dimethyl-5-formyl-1,3-dioxane was obtained as a pale yellow oil (1.8g.) and was used without further purification.

Nuclear magnetic resonance spectrum (NMR) was as follows: $^1$H (ppm from TMS in $CDC1_3$, integral, number of peaks, $J_{Hz}$):

9.80, 1H, s; 4.25, 2H, d, 12; 3.90, 2H, d, 12; 2.00 - 0.8, 17H, m.

Infrared spectrum (1R) (liquid film):

$\nu$ 1730 cm.$^{-1}$.

(vi) Methyl magnesium iodide (3.0ml, 3M solution in ether) was added to a solution of 5-cyclohexyl-2,2-dimethyl-5-formyl-1,3-dioxane (1.8g.) in dry ether (50ml.) The mixture was refluxed, with stirring, for two hours, cooled and poured into a mixture of 2N hydrochloric acid solution in ice. The aqueous mixture was extracted with ether. The ether extracts were washed with water, dried over anhydrous magnesium sulphate and evaporated in vacuo. 5-Cyclohexyl-2,2-dimethyl-5-(1-hydroxyethyl)-1,3-dioxane was obtained as a cream solid (1.5g.)

Gas-liquid chromatography (g.l.c.): OV210 at 170° produced one peak.

Nuclear magnetic resonance spectrum (NMR) was as follows: $^1$H (ppm from TMS in $CDC1_3$, integral, number of peaks, $J_{Hz}$):

4.15, 1H, q., 6; 3.90 - 3.50, 4H, m; 3.10, 1H, s broad; 2.00 - 0.9, 20H, m.

(vii) 5-Cyclohexyl-2,2-dimethyl-5-(1-hydroxyethyl)-1,3-dioxane (1.5g.) and Dowex 50x8-200 ion exchange resin ($H^+$ form) (1.0g.) in methanol (30ml.) and water (10ml.) was refluxed with stirring for six hours. The mixture was filtered and the filtrate was evaporated in vacuo. 2-Cyclohexyl-2-hydroxymethyl-butan-1,3-diol was obtained as a pale yellow oil (1.2g.) and was used without further purification.

Gas-liquid chromatography (g.l.c.): OV210 at 160° produced one peak.

Nuclear magnetic resonance spectrum (NMR) was as follows: $^1$H (ppm from TMS in $CDC1_3$, integral, number of peaks, $J_{Hz}$):

4.30, 1H, m; 4.00, 2H, s; 3.95, 2H, s; 3.20, 3H, s broad; 2.00 - 0.90, 14H, m.

(viii) From 2-cyclohexyl-2-hydroxymethyl-butan-1,3-diol (0.27g.) and trimethyl 4-bromo-orthobenzoate using the method described in Example A, 1-(4-bromophenyl)-4-cyclohexyl-3-methyl-2,6,7-trioxabicyclo-[2,2,2]octane, a colourless solid, was obtained (0.14g.)

Gas-liquid chromaography (g.l.c.): OV210 at 240° produced one peak.

1,4-Dicyclohexyl-3-methyl-2,6,7-trioxabicyclo[2,2,2]octane was prepared in an analogous manner by substituting trimethyl ortho-cyclohexyl-carboxylate for trimethyl 4-bromo-orthobenzoate in the final condensation reaction.

## Example D

### 2-(4-Bromophenyl)-4,4a,5,6,7,8,8a-heptahydro 2,4a-epoxymethanobenzo-1,3-dioxin

(i) A tetrahydrofuran solution (30ml) of ethylmagnesium bromide was prepared from bromoethane (7.05g.) and magnesium (1.5g.) The solution was cooled to 0° and 2-carbethoxycyclohexanone (10.0g.) was added carefully. The reaction mixture was stirred at room temperature for 30 minutes and ethyl chloroformate (7.0g.) was added dropwise, under nitrogen. A white precipitate developed. Sulphuric acid solution (80ml, 1%) was added and the aqueous mixture was extracted with ether. The ether extracts were washed with dilute aqueous sodium hydrogen carbonate solution, water and then dried over anhydrous magnesium sulphate. The solvent was removed in vacuo. Distillation gave 2,2-dicarbethoxycyclohexanone (7.8g.), a colourless oil, (b.pt. 98-102° 0.9 mm.)

Nuclear magnetic resonance spectrum (NMR) was as follows: $^1H$ (ppm from TMS in $CDC1_3$, integral, number of peaks, $J_{Hz}$):

4.3, 4H, q., 6; 2.5, 4H, m; 1.8, 4H, m; 1.4, 6H, t, 6.

(ii) A solution of 2,2-dicarbethoxycyclohexanone (3.0g.) in dry ether (15ml.) was added dropwise to a stirred suspension of lithium aluminium hydride (1.4g.) in dry ether (50ml.) at 0°, under nitrogen. The mixture was refluxed, with stirring, for six hours. Potassium hydroxide (6g.) in water (100ml) was added to the stirred mixture. Dipotassium hydrogen phosphate (4.2g.) and potassium dihydrogen phosphate (3.3g.) in water (50ml.) was added. Ether was removed by passing a current of nitrogen through the mixture. The resultant slurry was acidified with glacial acetic acid and the solid was filtered off. The filtrate was evaporated in vacuo. The residue was extracted with acetone and the solution was evaporated in vacuo. 2,2-Di-(hydroxymethyl)-cyclohexanol (1.2g.) was obtained as a pale yellow oil.

Gas-liquid chromatography (g.l.c.): OV210 at 150° produced one peak.

Nuclear magnetic resonance spectrum (NMR) was as follows: $^1H$ (ppm from TMS in $CDC1_3$, integral, number of peaks, $J_{Hz}$):

4.6, 3H, s; 4.00-3.70, 5H, m; 1.80-0.9, 8H,m.

(iii) 2,2-Di-(hydroxymethyl)-cyclohexanol (0.76g.) was added to trimethyl 4-bromo-orthobenzoate (1.1g.). One drop of concentrated hydrochloric acid was added and the mixture was heated at 140° for three hours. The volatile components were removed in vacuo (3.0m.m.) at 150°. The residue was purified by chromatography on alumina (alumina Woehm, TSC), eluting with 1:1 dichloromethane: hexane, saturated with ammonia.

2-(4-Bromophenyl)-4,4a,5,6,7,8,8a-heptahydro-2,4a-epoxymethanobenzo-1,3-dioxin was obtained as a colourless solid (0.27g.)

2-cyclohexyl-4,4a,5,6,7,8,8a-heptahydro-2,4a-epoxymethanobenzo-1,3-dioxin was prepared in analogous manner.

Example E

1-(4-Chlorophenyl)-3,5-dimethyl-4-ethyl-2,6,7-trioxabicyclo[2,2,2]octane

(i) 2,2-Dimethyl-5-ethyl-5-hydroxymethyl-1,3-dioxane (10g.) was added to a stirred suspension of sodium hydride (2.4g., 50% dispersion in oil) in dry tetrahydrofuran (80ml.) The mixture was refluxed with stirring for one hour. Benzyl chloride (9.2ml.) was added and the mixture was refluxed with stirring for three hours. the mixture was poured into water and the aqueous mixture was extracted with ether. The ethereal extract was washed with water, dried over anhydrous magnesium sulphate and evaporated in vacuo. 5-Benzyloxymethyl-2,2-dimethyl-5-ethyl-1,3-dioxanewas obtained as a pale brown oil (15g.) and was used without further purification.

(ii) 5-Benzyloxymethyl-2,2-dimethyl-5-ethyl-1,3-dioxane (15.0g.) and Dowex 50x8-200 ion exchange resin ($H^+$ form) (2.0g.) in methanol (250ml.) containing water (50ml.) was refluxed, with stirring, for three hours. The mixture was filtered and the filtrate was evaporated in vacuo. 2-Benzyloxymethyl-2-hydroxymethyl-butan-1-ol was obtained as a yellow oil (9.7g.).

Nuclear magnetic resonance spectrum (NMR)was as follows: $^1H$ (p.p.m. from TMS in $CDC1_3$, integral, number of peaks, $J_{Hz}$)

7.35, 5H, s; 4.55, 2H,s; 3.65,4H,s; 3.55,4H, s; 1.40, 2H, m; 0.90, 3H, t, 6.

(iii) 2-Benzyloxymethyl-2-hydroxymethyl-butan-1-ol (9.7g.) was added to a stirred suspension of pyridinium chlorochromate (9.7g.) and anhydrous sodium acetate (2.0g.) in dry dichloromethane (200ml.) at 0°, under a current of nitrogen. The mixture was stirred at room temperature for six hours. The mixture was diluted with dry ether (200ml.) and the organic solution was decanted off. The oily residue was treated with ether and the combined extracts were evaporated in vacuo. The residue was purified by chromatography on silica, eluting with 1:2 ether: hexane. 2-Benzyloxymethyl-2-ethyl-propandial was obtained as a pale brown oil (3.3g.) and was used immediately.

Infrared spectrum (liquid film):

$\nu$ 1720 $cm.^{-1}$

(iv) Methyl magnesium iodide (16ml. 3M solution in ether) was added to a solution of 2-benzyloxymethyl-2-ethylpropandial (3.2g.) in dry ether (70ml.). The mixture was refluxed, with stirring, for two hours and cooled. Aqueous ammonium chloride solution was added (30ml., 10%) and the aqueous mixture was extracted with ether. The ethereal extracts were washed with water, dried over anhydrous magnesium sulphate and evaporated in vacuo. The residue was purified by chromatography on silica, eluting with ether. 3-Benzyloxymethyl -3-ethyl-pentan-2,4-diol was obtained as a pale yellow oil(2.1g.).

Gas-liquid chromatography (g.l.c.): OV210 at 185° showed one peak.

Nuclear magnetic resonance spectrum (NMR) was as follows: $^1$H (p.p.m. from TMS in $CDC1_3$, integral, number of peaks):

7.40, 5H, s; 4.60, 2H, s; 4.40-3.40, 6H m; 1.60-0.75, 11H, m;

(v) 3-Ethyl-3-hydroxymethyl-pentan-2-4-diol was prepared from 3-benzyloxymethyl-3-ethyl-pentan-2,4-diol using the method described in stage (iv) of example C. 3-Ethyl-3-hydroxymethyl-pentan-2,4-diol was obtained as a pale brown oil.

Gas-liquid chromatography (g.l.c.): OV210 at 170° showed one peak.

Nuclear magnetic resonance spectrum (NMR) was as follows: $^1$H (p.p.m. from TMS in $CDC1_3$, integral, number of peaks):

4.60 - 3.50, 7H, m; 1.80-0.70, 11H, m.

(vi) 1-(4-Chlorophenyl)-3,5-dimethyl-4-ethyl-2,6,7-trioxabicyclo [2,2,2] octane (0.21g.), a colourless oil, was prepared from 3-ethyl-3-hydroxymethl-pentan-2,4-diol (0.7g.) using the method described in stage (iii) of example A.

Gas-liquid chromatography (g.l.c.): OV210 at 220° produced one peak

In an analogous manner the following compounds were prepared:-

1-cyclohexyl-3,5-di-methyl-4-n-propyl-2,6,7-trioxabicyclo[2,2,2]octane

1-(4-bromophenyl)-3,5-di-methyl-4-n-propyl-2,6,7-trioxabicyclo[2,2,2]octane

1-(4-chlorophenyl)-3,5-di-methyl-4-n-propyl-2,6,7-trioxabicyclo[2,2,2]octane

## Example F

### 1-(4-Chlorophenyl)-4-n-Propyl-3-trifluoromethyl-2,6,7-trioxabicyclo [2,2,2]octane

Sodium hydride (8.0g 60% dispersion in oil) was added to a stirred solution of diethyl n-propylmalonate (40g.) in dry benzene (200 ml.). The mixture was maintained at 60°, with stirring, for 1 hour. The mixture was cooled and trifluoracetic anhydride (28ml.) was added carefully. The mixture was stirred at room temperature for 2 hours. Water was added and the aqueous mixture was extracted with diethyl ether. The ethereal extracts were washed with water, dried over anhydrous magnesium sulphate and evaporated in vacuo.

Distillation gave diethyl 2-n-propyl-2-trifluoroacetylmalonate, a colourless oil (b.pt, 73°, 0.2m.m.) (35g.).

Gas-liquid chromatography (g.l.c.): OV 210 at 130° produced one peak.

Nuclear magnetic resonance spectrum (NMR) was as follows: $^1$H (ppm from TMS in $CDC1_3$, integral, number of peaks, $J_{H2}$):

4.30, 4H, q, 8; 2.00, 2H, m; 1.50 - 0.70, 11H,m.

3,3-Di-(hydroxymethyl)-1,1,1-trifluoro-hexan-2-ol was prepared from diethyl 2-n-propyl-2-trifluoroacetyl-malonate using the method described for the preparation of 2-ethyl-2-hydroxymethyl-butan-1,3-diol [example A(ii)].

1-(4-Chlorophenyl)-4-n-propyl-3-trifluoromethyl-2,6,7-trioxabicyclo[2,2,2]octane 1-cyclohexyl-4-n-propyl-3-trifluoromethyl-2,6,7-trioxabicyclo[2,2,2]octane, and 1-(4-bromophenyl)-4-n-propyl-3-trifluoromethyl-2,6,7-trioxabicyclo[2,2,2]octane were prepared from 3,3-di-(hydroxymethyl)-1,1,1-trifluorohexan-2-ol in an analogous method as in Example A (stage (ii).

Using similar methods 1-(4-chlorophenyl)-4-cyclohexyl-3-trifluoromethyl-2,6,7-trioxabicyclo[2,2,2]octane, 1,4-dicyclohexyl-3-trifluoromethyl-2,6,7-trioxabicyclo [2,2,2]octane and 1-(4-bromophenyl)-4-cyclohexyl-3-trifluoromethyl-2,6,7-trioxabicyclo[2,2,2]octane were prepared.

By replacing diethyl n-propylmalonate with diethyl prop-2-enylmalonate or diethyl 2-methylprop-2-enylmalonate the following compounds were prepared:

1-(4-bromophenyl)-4-prop-2-enyl-3-trifluoromethyl-2,6,7-trioxabicyclo[2,2,2]octane

1-cyclohexyl-4-prop-2-enyl-3-trifluoromethyl-2,6,7-trioxabicyclo[2,2,2]octane

1-(4-bromophenyl)-4-(2-methylprop-2-enyl)-3-trifluoromethyl-2,6,7-trioxabicyclo [2,2,2]octane

1-cyclohexyl-4-(2-methylprop-2-enyl)-3-trifluoromethyl-2,6,7-trioxabicyco[2,2,2]octane

## Example G

### 1-(4-Ethynylphenyl)-4-n-propyl-3-trifluoromethyl-2,6,7-trioxabicyclo[2,2,2]octane

(i) A mixture of 3,3-di-(hydroxymethyl)-1,1,1-trifluorohexan-2-ol (2.8g.), diethyl carbonate (1.6ml.), potassium hydroxide (0.1g) and dry ethanol (4.0 mls) was refluxed gently (oil bath 110°), under a current of

nitrogen, for 30 minutes. The ethanol was then removed by distillation. Distillation gave 3-(1-hydroxy-2,2,2-trifluoroethyl)-3-n-propyloxetane (1.7g.), a colourless oil (b.p. 112°, 20-25 m.m.).

Gas-liquid chromatography (g.l.c.): OV 210 at 120° produced one peak.

Infrared spectrum (1R) (liquid film):

3450 (s,br), 1300 (s), 1170(s), 1130(s), 1045 (s).

(ii) A solution of 4-iodobenzoyl chloride (2.1g.) in dry dichloromethane (25mls) was added to a stirred solution of 3-(1-hydroxy-2,2,2-trifluoroethyl)-3-n-propyl-oxetane (1.55g.) and pyridine (1.0ml.) in dry dichloromethane, at 0° The reaction mixture was stirred for 24 hours, at room temperature. The mixture was poured into water and the aqueous mixture was extracted with diethyl ether. The ethereal extracts were washed with water, dried over anhydrous magnesium sulphate and evaporated in vacuo. The residue was purified by column chromatography on silica, eluting with 1% triethylamine in hexane.

3-[1-(4-Iodobenzoyloxy)-2,2,2-trifluoroethy]-3-n-propyloxetane was obtained as a colourless oil (2.4g.).

Gas-liquid chromatogrophy (g.l.c.): OV 210 at 200° produced one peak.

Nuclear magnetic resonance spectrum (NMR) was as follows: $^1H$ (ppm from TMS in $CDC1_3$, integral, number of peaks):

7.70, 4H, m; 4.80 - 4.20, 5H, m; 2.20 - 0.80, 7H, m.

(iii) Boron trifluoride etherate (0.54ml.) was added to a stirred solution of 3-[1-(4-iodobenzoyloxy)-2,2,2-trifluoroethyl]-3-n-propyloxetane (2.3g.) in dry dichloromethane (50mls.) at -70°. The mixture was allowed to warm up slowly to room temperature and was then stirred for 12hours. Triethylamine (1.0ml.) was added and the mixture was poured into water. The aqueous mixture was extracted with diethyl ether. The ethereal extracts were washed with water, dried over anhydrous magnesium sulphate and evaporated in vacuo. The residue was purified by column chromatography on alumina eluting with 1 : 4 dichloromethane : hexane saturated with ammonia.

1-(4-Iodophenyl)-4-n-propyl-3-trifluoromethyl-2,6,7-trioxabicyclo(2,2,2)octane was obtained as a colourless solid (0.53g.).

Gas-liquid chromatography (g.l.c.): OV210 at 220° produced one peak.

Nuclear magnetic resonance spectrum (N.M.R.) was as follows : $^1H$ (ppm from TMS in $CDC1_3$, integral, number of peaks, $J_{Hz}$):

7.70, 2H, d, 8; 7.30, 2H, d, 8; 4.80 - 3.80, 5H, m; 1.40, 4H, m; 1.00, 3H, m

1-(4-Cyanophenyl)-4-n-propyl-3-trifluoromethyl-2,6,7-trioxabicyclo[2,2,2]octane was prepared in an analogous manner by substituting 4-cyanobenzoyl chloride for 4-iodobenzoyl chloride in step (ii)..

1-(4-Bromo-3,5-dichlorophenyl)-4-n-propyl-3-trifluoromethyl-2,6,7-trioxabicyclo [2,2,2]octane and 1-(4-bromo-3-chlorophenyl)-4-n-propyl-3-trifluoromethyl-2,6,7-trioxabicyclo[2,2,2] octane were prepared in an analogous manner by substituting 4-bromo-3,5-dichlorobenzoyl chloride and 4-bromo-3-chlorobenzoyl chloride respectively for 4-iodobenzoyl chloride in step (ii).

(iv) Bis-triphenylphosphine palladium dichloride (20mg.) and cuprous iodide (5mg.) were added to a stirred solution of 1-(4-iodophenyl)-4-n-propyl-3-trifluoromethyl-2,6,7-trioxabicyclo[2,2,2]octane (0.5g.) and trimethylsilyl-acetylene (0.24ml.) in dry diethylamine (20mls.). The mixture was stirred at room temperature for 12 hours. The mixture was evaporated in vacuo. and the residue was dIssolved in diethyl ether. The ethereal solution was washed with water, dried over anhydrous magnesium sulphate and evaporated in vacuo. The residue was purified by column chromatography on alumina eluting with 1 : 4 dichloromethane : hexane, saturated with ammonia. 4-n-Propyl-3-trifluoromethyl-1-[4-(2-trimethylsilylethynyl)-phenyl]-2,6,7-trioxabicyclo[2,2,2] octane was obtained as a pale brown solid (0.36g.).

Gas-liquid chromatography (g.l.c.): OV210 at 230° produced one peak.

(v) Tetrabutylammonium fluoride solution (0.84ml., 1M solution in tetrahydrofuran) was added to a stirred solution of 4-n-propyl-3-trifluoromethyl-1-[4-(2-trimethylsilylethynyl)phenyl]-2,6,7-trioxabicyclo[2,2,2]-octane (0.28g.) in dry tetrahydrofuran (5mls). The mixture was stirred for 30 minutes at room temperature. The solvent was removed in vacuo and the residue was dissolved in dry diethyl ether. The ethereal solution was washed with water, dried over anhydrous magnesium sulphate and evaporated in vacuo. The residue was recrystallised from hexane. 1-(4-Ethynylphenyl)-4-n-propyl-3-trifluoromethyl-2,6,7-trioxabicyclo[2,2,2]octane was obtained as a pale yellow solid (70mg.).

Gas-liquid chromatography (g.l.c.): 0V210 at 230° produced one peak.

Example H

1-(4-Ethylphenyl)-3-Methyl-4-Propyl-2,6,7-Trioxabicyclo[2,2,2]Octane.

(i) A mixture of 2,2-di-(hydroxymethyl)pentan-1-ol(24.6g), diethyl carbonate (20.1ml.), potassium hydroxide (0.3g.) and dry ethanol (2ml) was heated to gentle reflux (oil bath 110-120°) under a stream of nitrogen for 30 minutes. After this time the ethanol formed was removed by distillation at atmospheric pressure (oil bath 130 - 140°, still head temperature 76°). The pressure was reduced to 20 mm. Hg and the oil bath temperature adjusted to 230°. 3-hydroxymethyl-3-n-propyl oxetane distilled as a colourless liquid (16.7 gms, head temperature 120 - 126°).

Gas-liquid chromatography (g.l.c.) : OV-210 at 120° produced one peak. Nuclear magnetic resonance spectrum (N.M.R.) was as follows : $^1H$ (ppm from TMS in $CDC1_3$, integral, multiplicity):
4.35, 4H, s; 3.60, 2H, m; 1.8 - 0.7, 7H, m.

(ii) A solution of dimethyl sulphoxide (12 ml) in dry dichloromethane (4.0ml.) was added to a solution of oxalyl chloride (7.4 ml) in dichloromethane (25 ml) stirred at -70° under nitrogen. After the addition was complete the resulting mixture was stirred for a further 5 minutes at -70° before a solution of 3-hydroxymethyl-3-n-propyloxetane (10.0g) in dichloromethane (25 ml) was added, dropwise, over 10 minutes. The resulting mixture was allowed to stir for a further 30 minutes when neat triethylamine (54 ml) was added over approximately 30 minutes. The reaction mixture was allowed to warm to room temperature over 3 hours when it was poured into water. The organic phase was separated and the aqueous layer further extracted with fresh dichloromethane. The combined organic extracts were washed with dilute hydrochloric acid, saturated sodium bicarbonate and brine. The resulting organic phase was dried over anhydrous magnesium sulphate and evaporated in vacuo to give 3-formyl-3-n-propyloxetane-(10.5g) as a yellow oil.

Gas-liquid chromatography (g.l.c.): OV-210 at 120° produced one peak. Infrared spectrum (1R) (liquid film) : 1730 $cm^{-1}$.
Nuclear magnetic resonance spectrum (N.M.R.) was as follows : $^1H$ (ppm from TMS in $CDC1_3$, integral, multiplicity, $JH_z$ 9.0, iH, S; 4.90, 2H, d, 6; 4.60,2H, d, 6; 2.30 - 1.0, 7H, m.

(iii) A solution of 3-formyl-3-n-propyloxetane (10g) in dry ether (50ml) was added to a stirred solution of methyl magnesium iodide (0.078 moles) in ether (100 ml) at 0°C under nitrogen. The reaction mixture was allowed to warm to room temperature and stirred for a further 3 hours. After this time it was cooled to 0°C and sufficient saturated ammonium chloride solution was added slowly to dissolve the resulting precipitate. The organic phase was separated and the aqueous layer re-extracted with fresh ether. The combined organic extracts were washed with brine, dried over anhydrous magnesium sulphate and then evaporated in vacuo. The residue was purified by column chromatography on silica, gradient elution with hexane/ether mixtures. Elution with neat ether gave 3-(1-hydroxyethyl)-3-n-propyl oxetane as an oil (2.5g).

Gas-liquid chromatography (g.l.c.): OV-210 at 120° produced one peak.

Infrared spectrum (IR) (liquid film) : 3420 (s,br), 1470 (s), 1120 (s), 985 (s) $cm^{-1.}$

Nuclear magnetic resonance spectrum (N.M.R.) was as follows : $^1H$ (ppm from TMS in $CDC1_3$, integral, multiplicity, $J_{Hz}$) : 4.65, 2H, d, 6; 4.35, 2H, d, 6; 3.9, 1H, q 6; 1.15, 3H, d, 6; 1.9 - 0.8, 7H, m.

(iv) A solution of 4-iodobenzoyl chloride (3.5g) in dry ether (25ml) was added to a stirred solution of 3-(1-hydroxyethyl)-3-n-propyloxetane (1.7g) and pyridine (1.8ml) in ether (50ml) at 0°C. The resulting mixture was stirred for 24 hours at room temperature. After this time the mixture was washed with water and brine. The organic extracts were dried over anhydrous magnesium sulphate and then evaporated in vacuo. The residue was purified by column chromatography on silica (pre-eluted with 1% triethylamine in hexane) and eluted with hexane/ether mixtures. 3-[1-(4-Iodobenzoyloxy)ethyl]-3-n-propyloxetane was obtained as a colourless oil (1.8g).

Gas-liquid chromatography (g.l.c.): OV:210, programmed from 120° to 250° produced one peak

Infrared spectrum (IR)(liquid film) : 1730 (s), 1285 (s), 1115 (s),1020 (s), 770 (s)$cm^{-1}$.

Nuclear magnetic resonance spectrum (N.M.R.) was as follows : $^1H$ (ppm from TMS in $CDC1_3$, integral, multiplicity) 7.8, 4H, s; 5.4, 1H, q; 4.85 - 4.35, 4H, m; 1.9 - 0.8, 10H, m.

(v) 3-[1-(4-Iodobenzoyloxy)ethyl]-3-n-propyloxetane was converted to 1-(4-ethy nyiphenyl)-3-methyl-4-propyl-2,6,7-trioxabicyclo[2,2,2]octane by a method stric tly analogous to that described in example G steps (iii), (iv) and (v) and the corresponding intermediates isolated and characterised.

Gas liquid chromatography (g.l.c.) on the final product OV-17 at 240°C produced one peak.

Example I

4-t-Butyl-3-cyano-1-(4-ethynylphenyl)-2,6,7-trioxabicyclo[2,2,2]octane

(i) To a stirred solution of pyridinium chlorochromate (6.5g) in dry dichloromethane (50ml), under a nitrogen atmosphere was added 3-t-butyl-3-hydroxymethyl-oxetane (2.9g) in dichloromethane (10ml). The mixture was stirred overnight and dry diethyl ether (100ml) was added. The mixture was filtered through a column of fluorisil. The column was washed with further portions of ether (2 x 50ml). The combined filtrates were evaporated in vacuo. 3-t-butyl-3-formyl-oxetane was obtained as a colourless solid (2.3g).

Nuclear magnetic resonance spectrum (NMR) was as follows: $^1H$ (ppm from TMS in $CDC1_3$, integral, number of peaks):

9.90, 1H, s; 4.70-4.40 (4H, q.); 1.05, 9H, s.

(ii) To a stirred solution of 3-t-butyl-3-formyl-oxetane (1.0g) and 4-iodobenzoyl chloride (2.6g) in dry diethyl ether (35ml), under nitrogen, was added a solution of sodium cyanide (1.25g) in water (1.0ml). The mixture was stirred overnight. The mixture was poured into water (50 ml.) and the aqueous mixture was extracted with diethyl ether. The ethereal solution was dried over anhydrous sodium sulphate and then evaporated in vacuo. The residue was chromatographed on silica pre-treated with triethylamine eluting with hexane : chloroform 1:3. The 4-iodobenzoate of 3-t-butyl-3-(cyano-hydroxymethyl)-oxetane was obtained as a colourless solid (2.54g.). Nuclear magnetic resonance spectrum (NMR) was as follows : $^1H$ (ppm from TMS in $CDC1_3$, integral, number of peaks):

7.90-7.70, 4H, q; 5.80, 1H, s; 4.80-4.60, 4H, m; 1.10, 9H, s.

4-t-Butyl-3-cyano-1-[4-(2-trimethylsilylethynyl)-phenyl]-2,6,7-trioxabicyclo[2,2,2]octane was prepared from the 4-iodobenzoate of 3-t-butyl-3-(cyano-hydroxymethyl)-oxetane using the methodology described in Example G.

A mixture of 4-t-butyl-3-cyano-1-[4-(2-trimethylsilylethynyl)-phenyl]-2,6,7-trioxabicyclo[2,2,2]octane (1.3g.) and anhydrous potassium carbonate in dry methanol (125 ml.) was stirred under dry nitrogen for 2 hours. The resulting solution was evaporated in vacuo. The residue was taken up in diethyl ether and the ethereal solution was washed with water. The ethereal solution was dried over anhydrous sodium sulphate and evaporated in vacuo. The residue was purified by chromatography on alumina eluting with hexane: dichloromethane 4:1, saturated with ammonia. 4-t-butyl-3-cyano-1-(4-ethynylphenyl)-2,6,7-trioxabicyclo[2,2,2]octane was obtained as pale yellow needles (600 mg.).

From 3-formyl-3-isopropyl-oxetane and 4-cyanobenzoyl chloride and using the methodology described above, 3-cyano-1-(4-cyanophenyl)-4-isopropyl-2,6,7-trioxabicyclo[2,2,2]octane was prepared.

EXAMPLE J

1-(4-Cyanophenyl)-3-ethynyl-4-isopropyl-2,6,7-trioxabicyclo[2,2,2]octane.

1) To a stirred suspension of lithium acetylide ethylenediamine complex (2.76g.) in dry dioxane (25 ml.) at 0°, under nitrogen, was added a solution of 3-formyl-3-isopropyl-oxetane (2.56g.) in dry dioxane (10 ml.). The mixture was allowed to warm up to room temperature. After 3 hours n.m.r. analysis revealed that 3-formyl-3-isopropyl-oxetane was still present. A further portion of lithium acetylide ethylenediamine complex (3.0g.) was added and stirring was continued overnight. The mixture was poured into iced water and the aqueous mixture was extracted with diethyl ether. The ethereal extracts were washed with water and dried over anhydrous sodium sulphate. The ethereal solution was evaporated in vacuo. 3-(1-Hydroxy-prop-2-ynyl)-3-isopropyl-oxetane was obtained as an orange oil (0.8g.).

Nuclear magnetic resonance spectrum (NMR) was as follows: $^1H$ (ppm from TMS in $CDC1_3$, integral, number of peaks):

4.45, 1H,s; 4.70-4.40, 4H, m; 2.50, 1H, s; 2.5, 1H, s; 2.10, 1H, m; 1.10-1.00, 6H, m.

From 3-(1-Hydroxy-prop-2-ynyl)-3-isopropyl-oxetane and 4-cyanobenzoyl chloride and using methodology described in Example G. 1-(4-Cyanophenyl)-3-ethynyl-4-isopropyl-2,6,7-trioxabicyclo[2,2,2]octane was prepared.

The mass spectra, intra red spectra and nucleur magnetic resonance spectra data for the compounds described in the examples is given in Table 1.

References

1. S.M. McElvain and R.E. Stern J. Amer.Chem. Soc., 1955, 77, 4571
2. V.Gash J. Org.Chem., 1972, 37, 2197
3. S.M. McElvain and J.T. Venerable J. Amer. Chem. Soc., 1950, 72, 1661.

4. J.P. Ferris, B.G. Wright and C.C. Crawford J. Org. Chem., 1965, 30, 2367.

Table 1

| Number | $R^2$ | R | $R^1$ | mpt (°C) | Mass Sprectrum Chemical Ionisation M+1 | Infrared Spectrum | Method of Example |
|---|---|---|---|---|---|---|---|
| 1. | 4-Clphenyl | Et | Me | 48-50 | 269 | 1100(s), 1080(m), 1020(m) | A |
| 2. | 4-Brphenyl | Et | Me | 53-4 | 313 315 | 1105(s), 1080(w),1020(s), 1005(w) | A |
| 3. | c.hex. | Et | Me | oil | 241 | 1080(s),1010(s), 975(m) | A |
| 4. | 4-Clphenyl | Et | Et | 73-4 | 283 | 1110(s), 1100(s),1025(s),1010(s) | B |
| 5. | c.hex. | Et | Et | oil | 255 | 1080(s), 1020(s), 970(m) | B |
| 6. | 4-Clphenyl | n-pr | Me | 39-41 | 283 | 1110(s), 1025(s), 1000(s) | A |
| 7. | 4-Brphenyl | n-pr | Me | 40 | 327 329 | 1100(s),1080(w),1020(s),1000(m) | A |
| 8. | c.hex | n-pr | Me | oil | 255 | 1087(s), 1017(s), 975(m) | A |
| 9. | 4-Clphenyl | i-pr | Me | oil | 283 | 1100(s), 1075(w), 1020(s) | A |
| 10. | c.hex. | i-pr | Me | oil | 255 | 1090(w), 1055(m), 1020(s), 975(w) | A |
| 11. | 4-Brphenyl | $-CH_2CH_2CH_2CH_2-$ | | Solid | 325 327 | | D |
| 12. | c-hex | $-CH_2CH_2CH_2CH_2-$ | | 43-46 | 253 | 1080(m),1060(m),1015(s),970(s) | D |
| 13. | 4-Brphenyl | c.hex. | Me | solid | 367 369 | | C |
| 14. | 4-Brphenyl | t-Bu | Me | 104-113 | 341 343 | 1085(m), 1060(w), 1010(s) | B |
| 15. | 4-Brphenyl | t-Bu | Et | 122-128 | 355 357 | 1090(m), 1060(w), 1020(s) | B |
| 16. | c.hex | c.hex | Me | 55-56 | 295 | 1090(m), 1020(s) | C |
| 18. | 4-Clphenyl | t-Bu | Me | 96-98 | 297 | 1140(s),1100(s),1025(s) | B |
| 19. | 4-Brphenyl | nPr | $CH=CH_2$ | solid | 339 341 | 1620(w),1105(s),1020(s) | B |

| Number | $R^2$ | R | $R^1$ | mpt | Mass Sprectrum Chemical Ionisation M+1 | Infrared Spectrum | Method of Example |
|---|---|---|---|---|---|---|---|
| 20. | c.hex | | nPr | $CF_3$ | 48-49 | 309 1170(s), 1140(s),1090(m), 1030(s) | F |
| 21 | 4-BrPhenyl | nPr | $CF_3$ | 54-56 | 381, 383 | 1170(s),1135(m),1120(m),1050(m),1025(m) | F |
| 22 | 4-Clphenyl | nPr | $CF_3$ | 71-73 | 337 | 1170(s),1140(m),1120(m),1045(s),1020(s) | F |
| 23 | 4-Brphenyl | c.hex | $CF_3$ | solid | 421,423 | 1180(s),1145(s),1115(s),1045(m),1025(s) | F |
| 24 | 4-Clphenyl | c.hex | $CF_3$ | solid | 377 | 1170(s), 1100(s),1120(s) | F |
| 25 | 4-Ethynyl-phenyl | | nPr | $CF_3$ | solid | 3310(w),1172(s),1130(m),1115(m) | G |
| 26 | 4-[2-(trim-ethylsilyl)-ethynyl]-phenyl | | nPr | $CF_3$ | solid | | G |
| 27 | 4-Brphenyl | c.hex | $MeOCH_2$ | solid | 397, 399 | 1100(s), 1020(s) | A |
| 28 | 4-Iphenyl | nPr | $CF_3$ | solid | | 1180(s),1145(s),1120(s),1025(s) | G |
| 29 | 4-ethynyl-phenyl | | nPr | Me | solid | 273 | H |
| 30 | 4-[2-(trim-ethylsilyl)-ethynyl]-phenyl | | nPr | Me | solid | 345 | H |
| 31 | 4-Iphenyl | nPr | Me | 58-63° | 375 | 1110(s), 1020(s), 830(s) | H |

c.hex = cyclohexyl
t-Bu = tertiary butyl
n-Pr = normal propyl
i-Pr = iso propyl

| Number | $R^2$ | R | $R^1$ | mpt | Mass Sprectrum Chemical Ionisation M+1 | Infrared Spectrum | Method of Example |
|---|---|---|---|---|---|---|---|
| 35 | 4-Br-phenyl | iPr | nPr | waxy solid | 355 357 | 1105(s),1085(m),1020(s) | A |
| 36 | c.hex | iPr | nPr | oil | 283 | 1050(m),1020(s),980(m) | A |
| 37 | 4CN-phenyl | nPr | $CF_3$ | solid | 328 | 2250(m),1170(s),1140(s),1110(s),1050(s),1015(s) | G |
| 38 | 4-Br-phenyl | 2-methyl-prop-2-enyl | $CF_3$ | solid | 393 395 | 1185(s),1065(m),1110(s),1020(s), | F |
| 39 | c.hex | 2-methyl-prop-2-enyl | $CF_3$ | oil | 321 | 1660(w),1165(s),1120(m),1085(m),1030(m) | F |
| 40 | c.hex | c.hex | $CF_3$ | solid | 349 | 1180(s),1100(s),1030(s) | F |
| 41 | c.hex | prop-2-enyl | $CF_3$ | oil | 307 | 1650(w),1180(s),1140(m),1090(m),1030(m) | F |

| Number | $R^2$ | R | $R^1$ | mpt | Mass Sprectrum Chemical Ionisation M+1 | Infrared Spectrum | Method of Example |
|---|---|---|---|---|---|---|---|
| 42. | 4-Br-phenyl | prop-2-enyl | $CF_3$ | solid | 379 381 | 1650(w),1180(s),1140(m),1110(s),1020(s) | F |
| 43 | c.hex | t-Bu | Me | 69-71° | | 1055(s),1025(s) | B |
| 44 | 4-Br-phenyl | n-pr | Et | solid | 341 343 | 1105(s),1032(s),1020(s) | A |
| 45 | c.hex | n-pr | Et | oil | 269 | 1090(m),1062(m),1020(s) | A |
| 46 | 4-Br-phenyl | i-pr | Et | oily solid | 341 343 | 1110(m),1020(s) | |
| 47 | 4-Br-phenyl | n-pr | $MeOCH_2$ | solid | 357 359 | 1110(m),1020(s) | A |
| 48 | c,hex | n-pr | $MeOCH_2$ | oil | 285 | 1105(m),1080(m),1020(s) | A |
| 49 | 4-Br-phenyl | i-pr | $MeOCH_2$ | solid | 357 359 | 1140(m),1100(s),1000(s) | A |
| 50 | c.hex | i-pr | $MeOCH_2$ | oil | 285 | 1100(m),1055(m),1025(s) | A |
| 51 | 4-Br-phenyl | n-Bu | Me | | 341 343 | 1100(s),1010(s),990(m) | A |
| 52 | 4-Cl-phenyl | n-Bu | Me | | 297 | 1095(s),1015(s),990(m) | A |
| 53 | 4-CN-phenyl | i-pr | CN | 141-143° | 285 | | |
| 54 | 4-CN-phenyl | i-pr | C≡CH | 84-85° | 284 | | |
| 55 | 4-I-phenyl | t-Bu | CN | 155-157° | 400 | | |
| 56 | 4-[2-trim-ethylsilyl)-ethynyl]-phenyl | t-Bu | CN | | | | |
| 57 | 4-ethynyl-phenyl | t-Bu | CN | 123-125° | 298 | | |
| 58 | 4-Br-3,5-di-cl-phenyl | n-pr | $CF_3$ | solid | 449 451 | | |
| 59 | 4-Br-3-cl-phenyl | n-pr | $CF_3$ | solid | 415 417 | | |

| Number | $R^2$ | R | $R^1$ | mp(°C) | Mass Spectrum Chemical ionisation M+1 | Infrared spectrum | Method of example |
|---|---|---|---|---|---|---|---|
| 60 | 4-Br-phenyl | n-Bu | $CF_3$ | 50-52° | 395 397 | 1155(s),1125(m) 1100(m),1005(s) | F |
| 61 | 4-I-phenyl | n-Bu | Me | solid | 389 | | H |
| 62 | 4-[2-(trimethylsilyl)ethynyl]-phenyl | n-Bu | Me | solid | 359 | | H |
| 63 | 4-Ethynyl-phenyl | n-Bu | Me | solid | 287 | | H |
| 64 | cyclohexyl | t-Bu | CN | oil | | | I |
| 65 | 4-Br-phenyl | n-pr | CN | 116-118° | | | I |
| 66 | 4-I-phenyl | n-pr | CN | solid | | | I |
| 67 | 4-[2-(trimethylsilyl)ethynyl]-phenyl | n-pr | CN | solid | | | I |
| 68 | 4-Ethynyl-phenyl | n-pr | CN | solid | | | I |

| Number | Nuclear Magnetic Resonance Spectrum [$^1H$ carried out in $CDCl_3$ amd expressed as ppm from TMS, number of protons, number of peaks, $J_{Hz}$-(where appropriate)]. |
|---|---|
| 1. | 7.55, 2H, d, 8; 7.30, 2H, d, 8; 4.50-3.80, 5H,m; 1.70- 1.90, 5H, m; 0.95, 3H, t, 6. |
| 2. | 7.45, 4H, s; 4.4-4.0, 5H, m; 1.7-1.2, 5H, m; 0.9, 3H, t, 6 |
| 3. | 4.00, 5H, m; 2.00-0.6, 19H,m |
| 4. | 7.50, 2H, d, 8; 7.30, 2H, d, 8; 4.1-3.8, 5H, m; 2.0-0.8, 10H, m |
| 5. | 3.90, 5H, m; 2.0-0.9,21H,m |
| 6. | 7.40, 2H, d, 8; 7.65, 2H, d, 8; 4.4-3.9, 5H, m; 1.7-0.9, 10H, m |
| 7. | 7.40, 4H, s; 4.50-4.00,5H, m; 1.40, 3H, d, 6; 1.30, 4H, m; 1.00, 3H, m |
| 8. | 4.25-3.6, 5H, m; 2.00-0.8, 21H, m |
| 9. | 7.50, 2H, d, 7; 7.25, 2H, d, 7; 4.5-4.00, 5H, m; 1.80-1.20, 4H, m; 0.95, 6H, d, 6. |
| 10. | 4.25, 1H, q, 6; 4.0, 4H, s; 2.00-0.8, 21H, m. |
| 11. | 7.40, 4H, s; 4.55, 1H, dd, 8, 3; 4.00, 4H, m; 2.20-0.90, 8H, m. |
| 12. | 4.35, 1H, dd, 7, 3; 3.80, 4H, m; 2.2-0.8, 19H, m. |
| 13. | 7.40, 4H, s; 4.55, 1H, q, 6; 4.15, 4H, s; 2.00-0.80, 14H, m. |
| 14. | 7.40, 4H, s; 4.60-4.00, 5H, m; 1.55, 3H, d, 6; 1.00, 9H,s. |
| 15. | 7.40, 4H, s; 4.40-4.00, 5H, m; 2.00, 2H, m; 1.20, 3H, t, 6; 1.00, 9H, s |
| 16. | 4.30, 1H, q, 6; 4.00, 4H,s, 2.20-0.80, 25H, m. |
| 18. | 7.40, 2H, d, 8; 7.20, 2H, d, 8; 4.80-3.80, 5H, m; 1.60, 3H, d, 7; 1.00. 9H, s |
| 19. | 7.50, 4H, s; 6.20-5.20, 1H, m; 4.60, 2H, m; 4.30-3.90, 5H, m; 1.40-0.70, 7H, m. |
| 20. | 4.60 - 3.60, 5H, m; 2.20 - 0.80, 18H, m. |

| Number | Nuclear Magnetic Resonance Spectrum ($^1H$ carried out in $CDCl_3$ amd expressed as ppm from TMS, number of protons, number of peaks, $J_{Hz}$-(where appropriate)). |
|---|---|
| 21. | 7.50, 4H, m; 4.80 - 3.80, 5H, m; 1.70 - 0.80, 7H, m. |
| 22. | 7.45, 2H, d, 8; 7.35, 2H, d, 8; 4.60 - 3.80, 5H, m; 1.60 - 0.80, 7H, m. |
| 23. | 7.50, 4H, s; 4.70, 1H, m; 4.20, 4H, m; 2.00 - 0.90, 11H, m. |
| 24. | 7.50, 2H, d, 8; 7.30, 2H, d, 8; 4.80, 1H, m; 4.30, 4H, m; 2.00 - 0.90, 11H, m. |
| 25. | 7.40, 4H, m; 4.70 - 3.80, 5H, m; 3.05, 1H, s; 1.50 - 0.80, 7H, m. |
| 26. | 7.50, 4H, m; 4.80 - 3.90, 5H, m; 1.60 - 0.80, 7H, m; 0.40, 9H, s. |
| 27. | 7.50, 4H, s; 4.80 - 3.50, 10H, m; 2.20 - 1.00, 11H, m. |
| 28. | 7.40, 2H, d, 8; 7.30, 2H, d, 8; 4.70 - 3.80, 5H, m; 1.40, 4H, m; 1.00, 3H, m. |
| 29. | 7.40, 4H, m; 4.5 - 3.8, 5H, m; 3.0, 1H, s; 1.3, 3H, d, 6; 1.3 - 0.7, 7H, m. |
| 30. | 7.45, 4H, m; 4.4 - 3.9, 5H, m; 1.4, 3H, d, 6; 1.4 - 08, 7H, m, 0.3, 9H, s |
| 31. | 7.70, 2H, d, 8; 7.30, 2H, d, 8; 4.5 - 3.9, 5H, m; 1.45, 3H, d, 6; 1.7 - 0.8, 7H, m. |
| 35 | 7.40, 4H, s; 4.40 - 4.00, 5H, m; 2.00 - 0.75, 14H, m. |
| 36 | 4.30 - 3.70, 5H, m; 2.20 - 0.70, 25H, m. |
| 37 | 7.65, 4H, s; 4.80 - 3.80, 5H, m; 1.75 - 0.80, 7H, m. |
| 38. | 7.50, 4H, s; 5.05, 1H, s; 4.80, 1H, s; 4.60 - 4.00, 5H, m; 2.30, 1H, d, 14; 2.20, 1H, d, 14; 1.75, 3H, s. |
| 39. | 4.95, 1H, s; 4.72, 1H, s; 4.40 - 3.70, 5H, m; 2.17, 1H, d, 14; 2.07, 1H, d, 14;1.90 - 0.90, 14H, m. |
| 40 | 4.60 - 3.80, 5H, m; 2.00 - 0.80, 22H, m. |
| 41 | 5.65, 1H, m; 5.15, 2H, m; 4.60 - 3.75, 5H, m; 2.35 - 0.90, 13H, m. |
| 42 | 7.50, 4H, s; 5.65, 1H, m; 5.20, 2H, m; 4.60 - 4.00, 5H, m; 2.40 - 2.10, 2H, m. |

| Number | Nuclear Magnetic Resonance Spectrum ($^1H$ carried out in $CDCl_3$ amd expressed as ppm from TMS, number of protons, number of peaks, $J_{Hz}$-(where appropriate)). |
|---|---|
| 43. | 4.40 - 3.80, 5H, m; 2.0 - 0.95, 23H, m. |
| 44 | 7.40,4H,s; 4.20-3.80,5H, m; 1.70, 2H, m; 1.40-0.90, 10H, m. |
| 45 | 4.00-3.75, 5H, m; 2.00-0.80, 23H, m. |
| 46 | 7.40, 4H, s; 4.20, 5H, m; 1.80, 3H, m; 1.30, 3H, m; 1.00, 6H, d, 6 |
| 47 | 7.40, 4H, s; 4.40-4.00, 5H, m; 3.80, 2H, d; 3.40, 3H, s; 1.40, 4H, m; 1.00, 3H, m. |
| 48 | 4.40-4.00, 5H, m; 3.80, 2H, d; 3.55, 3H, s; 2.20-1.00, 18H, m. |
| 49 | 7.40, 4H, s; 4.50, 1H, m; 4.20, 4H, s; 3.80, 2H, d,(broad),3.40,3H,s;2.00,1H, m; 1.00,6H,dd. |
| 50 | 4.30, 1H, m; 4.00, 4H, m; 3.65, 2H, m; 3.40, 3H, s; 2.00-0.80, 18H, m |
| 51 | 7.50,4H,s; 4.50-3.90,5H,m; 1.50-0.90, 12H, m |
| 52 | 7.60, 2H, d,7: 7.40, 2H,d,7; 4.50-3.90, 5H, m; 1.50-0.90, 12H, m. |
| 53 | 7.65, 4H, q; 5.05, 1H, d; 4.40-4.10, 4H, m; 1.95, 1H, m; 1.00, 6H, m. |
| 54 | 7.75, 4H, q; 5.00, 1H, m; 4.40-4.00, 4H, m; 2.55, 1H, 2s; 1.90, 1H, m; 0.95, 6H, m. |
| 55 | 7.70-7.30, 4H, q; 4.95, 1H, d; 4.50-4.00, 4H, m; 1.05, 9H, s. |
| 56 | 7.60-7.40, 4H, q; 4.95, 1H, d; 4.50-4.00, 4H, m; 1.00, 9H, s; 0.20, 9H, s. |
| 57 | 7.60-7.40, 4H, q; 4.95, 1H, d; 4.50-4.00, 4H, m; 3.05, 1H, s; 1.05, 9H, s. |
| 58 | 7.60,2H,s; 4.50,1H,m; 4.35,1H m; 4.10,3H,m; 1.60-0.85, 7H,m. |
| 59 | 7.70, 1H,d,1.5; 7.60,1H,d,7; 7.35,1H,dd,7, 1.5; 4.50, 1H,m; 4.35, 1H,m; 4.05, 3H,m; 1.60-0.80, 7H,m. |

60 7.50,4H,s; 4.50,1H,m; 4.35,1H,m; 4.05,3H,m; 1.50-0.80,9H,m.

61 7.70,2H,d,7; 7.40,2H,d,7; 4.60-4.00,5H,m; 1.80-0.80,12H,m.

62 7.55,2H,d,7; 7.45,2H,d,7; 4.40-3.90,5H,m; 1.40,3H,d,6; 1.30,6H,m; 0.90,3H,t,6; 0.25,9H,s.

63 7.55,2H,d,7; 7.50,2H,d,7; 4.35,1H,m; 4.25-3.95,4H,m; 3.05,1H,s; 1.50-1.10,9H,m; 0.95,3H,t,7.

64 4.75,1H,d; 4.30-3.75,4H,m; 1.85-1.50,6H,m; 1.20-1.00,5H,m; 0.95,9H,s.

65 7.45,4H,m; 4.90,1H,d; 4.40-4.05,4H,m; 1.45-1.20,4H,m; 0.95-3H,t.

66 7.70,2H,d; 7.30,2H,d; 4.90,1H,d; 4.35-4.05,4H,m; 1.45-1.15,4H,m; 0.95,3H,t.

67 7.50,2H,d; 7.45,2H,d; 4.90,1H,d; 4.40-4.05,4H,m; 1.50-1.10,4H,m; 0.95-3H,t; 0.20,9H,s.

68 7.50,2H,d; 7.45,2H,d; 4.95,1H,d; 4.35-4.05,4H,m; 3.05-1H,s; 1.45-1.10,4H,m; 0.95,3H,t.

| Number | $R^2$ | R | $R^1$ | $R^3$ | mpt | Mass Sprectrum Chemical Ionisation M+1 | Infrared Spectrum | Method of Example |
|---|---|---|---|---|---|---|---|---|
| 17 | 4-Clphenyl | Et | Me | Me | oil | 283 | 1105(s),1080(w),1025(s),1000(s) | E |
| 32 | 4-Brphenyl | nPr | Me | Me | oil | 341,343 | | E |
| 33 | 4-Clphenyl | nPr | Me | Me | oil | 297 | 1100(s),1027(s) | E |
| 34 | c.hex | nPr | Me | Me | oil | 269 | 1100(s),1020(s) | E |

| Number | Nuclear Magnetic Resonance Spectrum |
|---|---|
| 17 | 7.45,2H,d,B; 7.20,2H,d,8; 4.40, 2H,q, 6; 4.00, 2H, m; 1.30, 8H, m; 0.90,3H, m. |
| 32 | 7.50,4H, s; 4.50-3.50, 4H, m; 1.70-0.80, 13H, m. |
| 33 | 7.45, 2H, d, 8; 7.35, 2H,d,8; 4.50-3.90, 4H, m; 1.50 -1.10, 10H, m; 0.90, 3H, m. |
| 34 | 4.30-3.70, 4H, m; 2.00-1.00, 21H, m; 0.90, 3H, m. |

## BIOLOGICAL ACTIVITY

The compound number refers to the numbers allocated to the compounds in the table spanning pages 41 to 44.

### A. Lethal activity against House flies

The activity of compounds of the invention against unanaesthatised female Musca domestica (WRL strain), was demonstrated by the topical application to the test insect of a solution of the compound under test in butanone.

The activity of the test compound was also assessed when applied topically in conjunction with a synergist [6μg piperonyl butoxide (PB) per insect]. Mortality was assessed after 24 and 48 hrs.

The following compounds were active at less than 30 μg/fly:
1,11,12,15,16,19,20,22,37,38,40,41,42,44,45,46,47,48,49,50,17,32,33,34.

The following compounds were active at less than 1 µg/fly: 6,7,8,9,10,13,14,18,21,23,24,25,26,27,29,30.

B. Lethal Activity Against Blattella germanica

The activity of oompounds of the invention against unaesthetised male Blatella germanica (WRL strain) was demonstrated by the topical application to the test insect of a solution of the compound under test in butanone.

The activity of the test compound was assessed when applied topically in conjunction with a synergist [10µg piperonyl butoxide (PB) per insect]. Mortality was assessed after 24 and 48 hours.

The following compounds were active at less than 50 µg/insect: 1,5,11,12,13,15,18,19,20,21,23,25,26,27,36,37,38,39,40,41,42,44,48,32,33,34.

The collowing compounds were active at less than 5µg/insect: 6,7,8,9,29,30.

C. Lethal Activity Against Sitophilus granarius

The activity of the compounds of the invention against S.granarius adults was demonstrated by addition of the compound in acetone solution to grain, to which the insects were later infested. Mortality was assessed after 6 days

The following compounds gave activity at less than 200 ppm solution of acetone: 16,18,19,20,22,23,216,27,37,41,42,43,44,32,33,34.

The following compounds gave activity at less than 50 ppm solution of acetone: 6,14,15,21,25,29.

D. Lethal activity against Culex quinquefasciatus

The activity of the compounds of the invention against female Culex adults was demonstrated by direct spraying of 0.5 ml of compound in OPD/Methylene chloride. Mortality was assessed after 24 hours.

The following compounds were active at less than 1.0%: 6,12,14,15,18,19,21,22,23,24,25,26,27,37,39,41,43,44,48,50,33.

The following compound was active at less than 0.1%: 29.

E. Mammalian Toxicity

Compound 15 has an $LD_{50}$ value of greater than 20mg/Kg when given orally to mice (Charles River CD1).

Formulations

| 1. Emulsifiable Concentrate | |
|---|---|
| Compound 11 | 10.00 |
| Ethylan KEO | 20.00 |
| Xylene | 67.50 |
| Butylated Hydroxyanisole | 2.50 |
| | 100.00 |

| 2. Wettable Powder | |
|---|---|
| Compound 11 | 25.0 |
| Attapulgite | 69.50 |
| Sodium isapropylbenzene sulphonate | 0.50 |
| Sodium salt of condensed naphthalene sulphonic acid | 2.50 |
| Butylated hydroxytoluene | 2.50 |
| | 100.00 |

| 3. Dust | |
|---|---|
| Compound 11 | 0.50 |
| Butylated Hydroxyanisole | 0.10 |
| Talc | 99.40 |
| | 100.00 |

| 4. Bait | |
|---|---|
| Compound 11 | 40.25 |
| Icing Sugar | 59.65 |
| Butylated hydroxy toluene | 0.10 |
| | 100.00 |

| 5. Lacquer | |
|---|---|
| Compound 11 | 2.5 |
| Resin | 5.0 |
| Butylated Hydroxy anisole | 0.5 |
| High aromatic white spirit | 92.0 |
| | 100.00 |

| 6. Aerosol | |
|---|---|
| Compound 11 | 0.30 |
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

| 7. Spray | |
|---|---|
| Compound 11 | 0.1 |
| Butylated Hydroxy anisole | 0.1 |
| Xylene | 10.0 |
| Odourless Kerosene | 89.8 |
| | 100.00 |

| 8. Potentiated Spray | |
|---|---|
| Compound 11 | 0.1 |
| Piperonyl Butoxide | 0.5 |
| Butylated Hydroxyanisole | 0.1 |
| Xylene | 10.1 |
| Odourless Kerosene | 89.2 |
| | 100.0 |

SCHEME 1

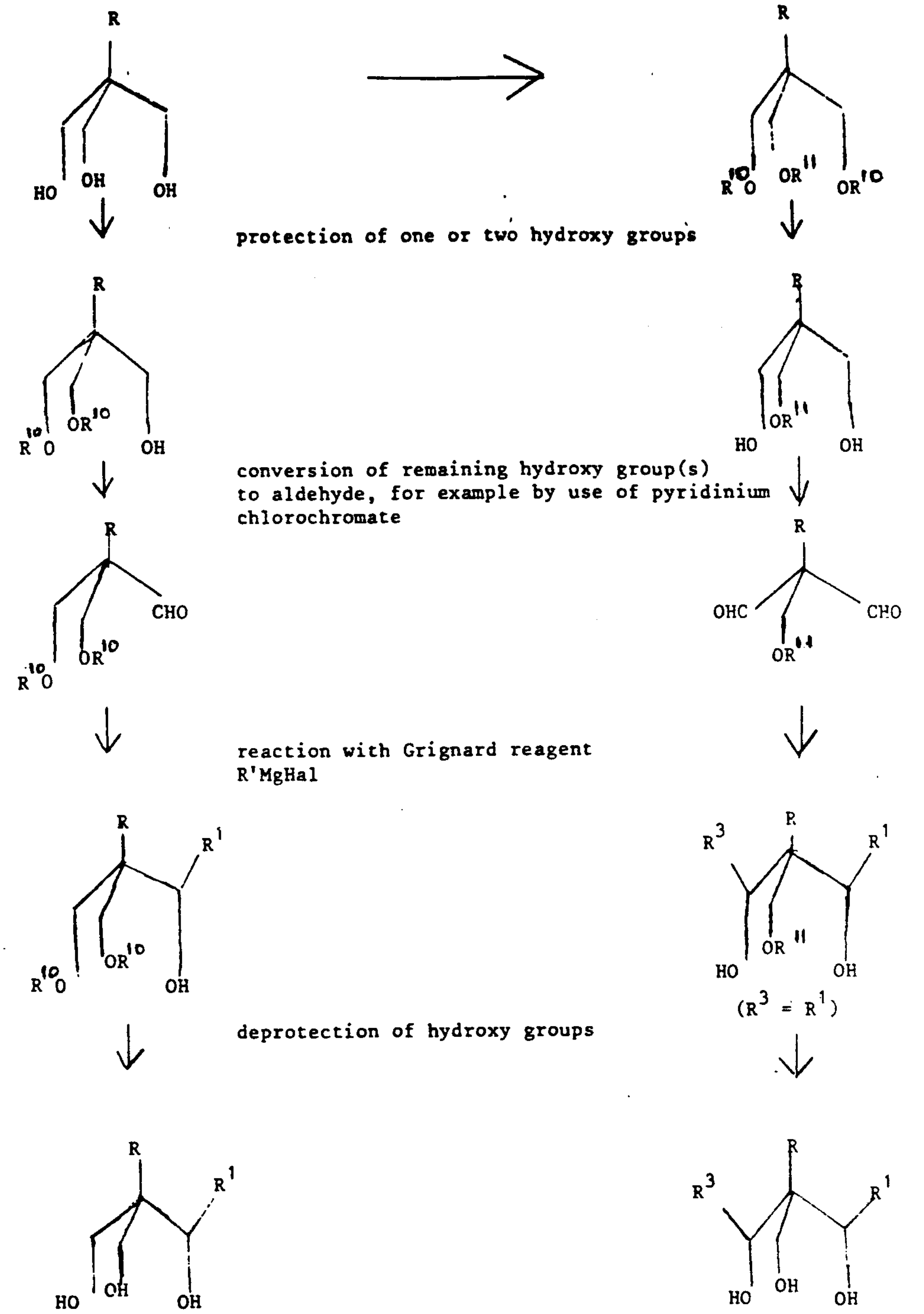


wherein $R^{10}$, $R^{11}$ are protecting groups that may be removed under different conditions, e.g. by hydrogenolysis and acid hydrolysis. Conveniently both groups $R^{10}$ are linked to form an isopropylidene group and $R^{11}$ is benzyl.

## Claims

**1.** A compound of the formula (I):

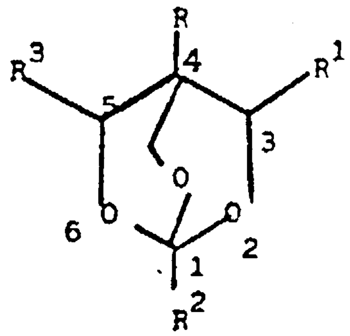

(I)

wherein R is $C_{2-10}$ alkyl, alkenyl or alkynyl, each optionally substituted by, or methyl substituted by, cyano, $C_{3-4}$ cycloalkyl,halo, $C_{1-4}$ alkoxy or a group S(O)m $R^4$ where $R^4$ is $C_{1-4}$ alkyl and m is 0, 1 or 2, or Ris $C_{3-10}$ cycloalkyl, $C_{4-10}$ cycloalkenyl or phenyl, each optionally substituted by $C_{1-4}$ alkoxy, $C_{1-3}$ alkyl, $C_{2-4}$ alkynyl,halo, cyano or a group S(O)m $R^4$ as defined hereinbefore; $R^1$ is halo, $C_{1-3}$ alkyl, $C_{2-3}$ alkenyl or alkynyl each optionally substituted by halo, cyano, $C_{1-4}$ alkoxy, alkyl carbalkoxy containing up to 6 carbon atoms, a group S(O)m $R^4$ as defined hereinbefore or alkynyl substituted by tri-$C_{1-4}$ alkylsilyl, or $R^1$ is cyano, spiro-cyclopropyl, gem dimethyl, gem dicyano, gem diethynyl, oxo or methylene optionally substituted by cyano or $C_{1-3}$ alkyl optionally substituted by fluorine, or $R^1$ and R and the carbon atoms to which they are attached form a $C_{5-7}$ carbocyclic ring optionally substituted by halo, $C_{1-3}$ alkyl or alkoxy or $C_{2-3}$ alkenyl, $R^2$ is phenyl, $C_{5-10}$ cycloalkyl or cycloalkenyl each optionally substituted by halo, cyano, azido, tetrazolyl, a group $SO_2R^5$ wherein $R^5$ is amino or di-$C_{1-4}$ alkylamino, a group $COR^6$ wherein $R^6$ is $c_{1-4}$ alkoxy, benzyloxy, amino or di-$C_{1-4}$ alkylamino, nitro, $C_{1-3}$ alkyl optionally substituted by halo, cyano or ethynl, or $C_{2-3}$ alkenyl or ethynyl each optionally substituted by halo or tri-$C_{1-4}$ alkylsilyl, and $R^3$ is hydrogen, $C_{1-3}$ alkyl, $C_2$-$_3$ alkenyl or alkynyl each optionally substituted by cyano, $c_{1-4}$ alkylthio, $C_{1-4}$ alkoxy or halo, or $R^3$ is cyano or halo.

**2.** A compound according to claim 1 in which R is n-propyl, n-butyl, i-butyl, s-butyl, t-butyl, cyclopentyl or cyclohexyl.

**3.** A compound according to either claim 1 or 2 in which $R^1$ is methyl, ethyl, trifluoromethyl, cyano or ethynyl.

**4.** A compound according to any one of claims 1 to 3 in which $R^2$ is cyclohexyl, cycloheptyl, cyclooctyl or phenyl optionally substituted at the 3-, 4- and/or 5-position by halo, cyano, ethynyl, azido or nitro and/or at the 2- and/or 6-position by fluoro.

**5.** A compound according to claim 4 in which $R^2$ is phenyl substituted of the 4-position by chlorine, bromine or ethynyi.

**6.** A compound according to any one of claims 1 to 5 in which $R^3$ is hydrogen or methyl.

**7.** A compound of the formula IA:

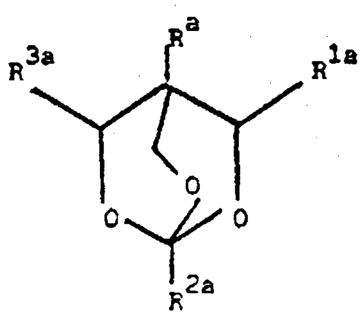

(IA)

wherein $R^a$ is $C_{2-4}$ alkyl, alkenyl or alkynyl, $C_{5-10}$ cycloaklyl or phenyl, each optionally substituted by cyano or $C_{1-4}$ alkoxy, $R^{1a}$ is cyano or $C_{1-3}$ alkyl, $C_{2-3}$ alkenyl or alkynyl each optionally substituted by cyano, $C_{1-4}$ alkoxy, $C_{1-4}$ alkylthio or halo,or $R^{1a}$ is cyano, gem dimethyl or $R^{1a}$ and $R^8$ and the carbon atoms to which they are attached form a $C_{5-7}$ carbocyclic ring optionally substituted by $C_{1-3}$ alkyl or alkoxy; $R^{2a}$ is phenyl, $C_{5-10}$ cycloaklyl or cycloalkenyl each optionally substituted and $R^{3a}$ is hydrogen, $C_{1-3}$ alkyl, $C_{2-3}$ alkenyl or alkynyl each optionally substituted by cyano, $C_{1-4}$ alkylthio, $C_{1-4}$ alkoxy or halo.

**8.** 1-(4-bromophenyl)-4-cyclohexyl-3-methyl-2,6,7-trioxabicyclo[2,2,2]octane,
1-(4-chlorophenyl)-3-methyl-4-iso-propyl-2,6,7-trioxabicyclo[2,2,2]octane,
1-(4-bromophenyl)-3-methyl-4-n-propyl-2,6,7-trioxabicyclo[2,2,2]octane,
1-(4-chlorophenyl)-3-methyl-4-n-propyl-2,6,7-trioxabicyclo[2,2,2]octane,
1-cyclohexyl-3-methyl-4-iso-propyl-2,6,7-trioxabicyclo[2,2,2]octane,
1-(4-bromophenyl)-4-t-butyl-3-methyl-2,6,7-trioxabicyclo[2,2,2]octane,
1,4-dicyclohexyl-3-methyl-2,6,7-trioxabicyclo[2,2,2]octane,
4-t-butyl-1-(4-chlorophenyl)-3-methyl-2,67-trioxabicyclo[2,2,2]octane,
1-(4-ethynylphenyl)-3-methyl-4-n-propyl-2,6,7-trioxabicyclo[2,2,2]octane,
3-methyl-4-n-propyl-1-[4-(2-trimethylsilylethynyl)-phenyl]-2,6,7-trioxabicyclo[2,2,2]octane,
1-cyclohexyl-3-methyl-4-n-propyl-2,6,7-trioxabicyclo[2,2,2]octane,
4-t-butyl-1-cyclohexyl-3-methyl-2,6,7-trioxabicyclo[2,2,2]octane,
1-(4-bromophenyl)-4-n-butyl-3-methyl-2,6,7-trioxabicyclo[2,2,2]octane,
4-n-butyl-1-(4-chlorophenyl)-3-methyl-2,6,7-trioxabicyclo[2,2,2]octane
4-n-Butyl-1-(4-indophenyl)-3-methyl-2,6,7-trioxabicyclo[2.2.2]octane,
4-n-Butyl-3-methyl-1-[4-(2-trimethylsilyl ethynyl)phenyl]-2,6,7-trioxabicyclo[2.2.2]octane, or
4-n-Butyl-1-(4-ethynyl phenyl)-3-methyl-2,6,7-trioxabicyclo[2.2.2]octane.

**9.** 1-(4-ethynylphenyl)-4-n-propyl-3-trifluoromethyl-2,6,7-trioxabicyclo[2,2,2]octane,
4-n-propyl-3-trifluoromethyl-1-[4-(2-trimethylsilylethynyl)-phenyl]-2,6,7-trioxabicyclo[2,2,2]octane,
1-(4-chlorophenyl)-4-n-propyl-3-trifluoromethyl-2,6,7-trioxabicyclo[2,2,2]octane,
1-(4-bromophenyl)-4-n-propyl-3-trifluoromethyl-2,6,7-trioxabicyclo[2,2,2]octane,
1-(4-chlorophenyl)-4-cyclohexyl-3-trifluoromethyl-2,6,7-trioxabicyclo[2,2,2]octane,
1-(4-bromophenyl)-4-cyclohexyl-3-trifluoromethyl-2,6,7-trioxabicyclo[2,2,2]octane,
1-cyclohexyl-4-n-propyl-3-trifluoromethyl-2,6,7-trioxabicyclo[2,2,2]octane,
1-(4-cyanophenyl)-4-n-propyl-3-trifluoromethyl-2,6,7-trioxabicyclo[2,2,2]octane,
1-(4-bromo-3,5-dichlorophenyl)-4-n-propyl-3-trifluoromethyl-2,6,7-trioxabicyclo[2,2,2]-octane or
1-(4-bromo-3-chlorophenyl)-4-n-propyl-3-trifluoromethyl-2,6,7-trioxabicyclo [2,2,2]-octane, or
4-n-Butyl-1-(4-bromophenyl)-3-trifluoromethyl-2,6,7-trioxabicyclo[2.2.2]octane.

**10.** 1-(4-bromophenyl)-3-ethenyl-4-n-propyl-2,6,7-trioxabicyclo[2,2,2]octane, or or 1-(4-cyanophenyl)-3-ethynyl-4-isopropyl-2,6,7-trioxabicyclo[2,2,2]octane

**11.** 1-(4-bromophenyl)-3-cyano-4-n-propyl-2,6,7-trioxabicyclo[2,2,2]octane,
3-cyano-1-(4-ethynylphenyl)-4-n-propyl-2,6,7-trioxabicylclo[2,2,2]octane,
4-t-butyl-3-cyano-1-(4-ethynylphenyl)-2,6,7-trioxabicyclo[2,2,2[octane

3-cyano-1-(4-cyanophenyl)-4-isopropyl-2,6,7-trioxabicylclo[2,2,2]octane,
4-t-butyl-3-cyano-1-(4-iodophenyl)-2,6,7-trioxabicyclo[2,2,2[octane
4-t-butyl-3-cyano-1-[4-(2-trimethylsilylethynyl)-phenyl)-2,6,7-trioxabicylclo[2,2,2,] octane,
4-t-Butyl-3-cyano-1-cyclohexyl-2,6,7-trioxabicyclo[2.2.2]octane,
1-(4-Bromophenyl)-3-cyano-4-n-propyl-2,6,7-trioxabicyclo[2.2.2]octane,
3-Cyano-1-(4-iodophenyl)-4-n-propyl-2,6,7-trioxabicyclo[2.2.2]octane,
3-Cyano-4-n-propyl-1-[4-(2-trimethylsilylethynyl)phenyl]2,6,7-trioxabicyclo[2.2.2]octane, or
3-Cyano-1-(4-ethynylphenyl)-4-n-propyl-2,6,7-trioxabicyclo-[2.2.2]octane.

**12.** 1-(4-bromophenyl)-4-cyclohexyl-3-methoxymethyl-2,6,7-trioxabicylclo[2,2,2]octane.

**13.** 1-(4-bromophenyl)-4-t-butyl-3-ethyl-2,6,7-trioxabicyclo[2,2,2]octane.

**14.** 1-(4-chlorophenyl)-3,5-dimethyl-4-n-propyl-2,6,7-trioxabicyclo[2,2,2]octane,
1-(4-bromophenyl)-3,5-dimethyl-4-n-propyl-2,6,7-trioxabicyclo]2,2,2]octane, or
1-cyclohexyl-3,5-dimethyl-4-n-propyl-2,6,7-trioxabicyclo[2,2,2]octane,

**15.** A process for the preparation of a compound of the formula (I) as defined in any one of claims 1 to 14 which comprises the condensation of an orthocarboxylate of the formula $R^2$ $C(OR^7)_3$ with a triol of the formula (II)

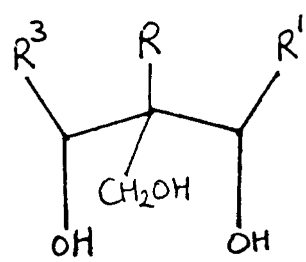

II

wherein R to $R^3$ are as hereinbefore defined and $R^7$ is $C_{1-4}$ alkyl, phenyl or $C_{7-8}$ aralkyl.

**16.** An insecticidal or acaricidal composition comprising a compound of formula (I) as defined in any one of claims 1-14 in admixture with a carrier or diluent.

**17.** A synergised pesticidal composition comprising a compound of formula (I), as defined in any one of claims 1-14, a synergist for the formula I compound and a carrier or diluent.

**18.** A mixture of a compound of formula (I) as defined in any one of claims 1-14 and another pesticidal compound.

**19.** A method for the control of pests comprising application to the pest or to an environment susceptible to pest infestation of a compound according to any one of claims 1-14 or a composition or mixture according to any one of claims 16-18.

**20.** A compound as defined in any one of claims 1-14 or composition according to any one of claims 16-18 for use in a method of surgery or therapy practised on the human or animal body or in a method of diagnosis practised on the human or animal body.

21. A compound of the formula:

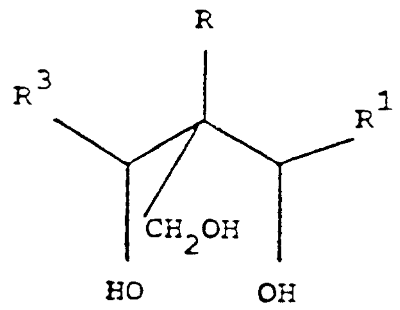

II

wherein R, $R^1$ and $R^3$ are as defined in any one of claims 1-3, or 6 to 14, with the proviso that when $R^1$ is $CH_3$ and $R^3$ is H, then R is not ethyl, isopropyl or n-propyl.

22. A compound according to claim 21 wherein when $R^1$ is $CH_3$ and $R^3$ is H, then R is not a $C_1$-$C_3$ alkyl group.

23. A compound according to claim 21 wherein R is $CF_3$.

**Patentansprüche**

1. Verbindung der Formel (I):

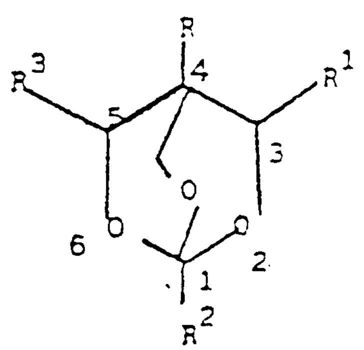

(I)

worin R C2-10-Alkyl, -Alkenyl oder -Alkinyl bedeutet, gegebenenfalls substituiert durch Cyan, C3-4-Cycloalkyl, Halogen, C1-4-Alkoxy oder eine Gruppe S(O)m R4 worin R4 C1-4-Alkyl und m 0, 1 oder 2 bedeutet, oder durch Methyl, das durch die vorstehend genannten Gruppen substituiert ist, oder R C3-10-Cycloalkyl, C4-10-Cycloalkenyl oder Phenyl ist, gegebenenfalls substituiert durch C1-4-Alkoxy, C1-3-Alkyl, C2-4-Alkinyl, Halogen, Cyan oder eine Gruppe S(O)m R4 wie vorstehend definiert, R1 Halogen, C1-3-Alkyl, C2-3-Alkenyl oder -Alkinyl ist, gegebenenfalls substituiert durch Halogen, Cyano, C1-4-Alkoxy, Alkylcarbalkoxy mit bis zu 6 Kohlenstoffatomen, eine Gruppe S(O)m R4 wie vorstehend definiert, oder durch tri-C1-4-Alkylsilyl substituiertes Alkinyl oder R1 Cyan, Spiro-Cyclopropyl, gem-Dimethyl, gem-Dicyano, gem-Diethinyl, Oxo oder Methylen ist, gegebenenfalls substituiert durch Cyan oder C1-3-Alkyl, das gegebenenfalls durch Fluor substituiert sein kann, oder R1 und R und die Kohlenstoffatome, an die sie gebunden sind, einen C5-7-carbocyclischen Ring bilden, gegebenenfalls substituiert durch Halogen, C1-3-Alkyl oder -Alkoxy oder C2-3-Alkenyl, R2 Phenyl, C5-10-Cycloalkyl oder -Cycloalkenyl ist, gegebenenfalls substituiert durch Halogen, Cyan, Azido, Tetrazolyl, eine Gruppe SO2R5, worin R5 Amino oder di-C1-4-Alkylamino bedeutet, eine Gruppe COR6, worin R6 C1-4-Alkoxy, Benzyloxy, Amino oder Di-C1-4-Alkylamino, Nitro, C1-3-Alkyl, gegebenenfalls substituiert durch Halogen, Cyan oder Ethinyl, oder C2-3-Alkenyl oder -Ethinyl, gegebenenfalls substituiert durch Halogen oder Tri-C1-4-Alkylsilyl, bedeutet, und R3 Wasserstoff, C1-3-Alkyl, C2-3-Alkenyl oder Alkinyl ist, gegebenenfalls substituiert durch Cyan, C1-4-Alkylthio, C1-4-Alkoxy oder Halogen, oder R3 ist Cyan oder Halogen.

2. Verbindung nach Anspruch 1, dadurch gekennzeichnet, daß R n-Propyl, n-Butyl, i-Butyl, s-Butyl, t-Butyl, Cyclopentyl oder Cyclohexyl ist.

3. Verbindung nach Anspruch 1 oder 2, dadurch gekennzeichnet, daß R1 Methyl, Ethyl, Trifluormethyl, Cyan oder Ethinyl ist.

4. Verbindung nach einem der Ansprüche 1 bis 3, dadurch gekennzeichnet, daß R2 Cyclohexyl, Cycloheptyl, Cyclooctyl oder Phenyl ist, die gegebenenfalls in der 3-, 4-und/oder 5-stellung durch Halogen, Cyan, Ethinyl, Azido oder Nitro und/oder an der 2- und/oder 6-Position durch Fluor substituiert sein können.

5. Verbindung nach Anspruch 4, dadurch gekennzeichnet, daS R2 ein in 4-stellung durch Chlor, Brom oder Ethinyl substituiertes Phenyl ist.

6. Verbindung nach einem der Ansprüche 1 bis 5, dadurch gekennzeichnet, daß R3 Wasserstoff oder Methyl ist.

7. Verbindung der Formel IA:

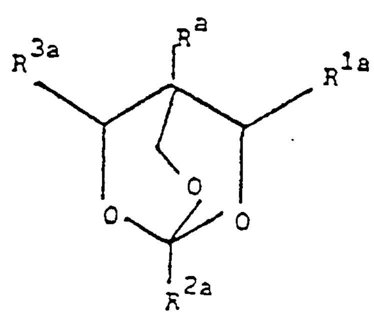

(IA)

worin $R^a$ C2-4-Alkyl, -Alkenyl oder -Alkinyl, C5-10-Cycloalkyl oder Phenyl ist, gegebenenfalls substituiert durch Cyan oder C1-4-Alkoxy, $R^{1a}$ ist Cyan oder C1-3-Alkyl, C2-3-Alkenyl oder -Alkinyl, gegebenenfalls substituiert durch Cyano, C1-4-Alkoxy, C1-4-Alkylthio oder Halogen, oder $R^{1A}$ ist Cyan, gem-Dimethyl oder $R^{1a}$ und $R^a$ und die Kohlenstoffatome, an die sie gebunden sind, bilden einen C5-7-carbocyclischen Ring, der gegebenenfalls durch C1-3-Alkyl oder -Alkoxy substituiert sein kann; $R^{2a}$ ist Phenyl , C5-10-Cycloalkyl oder Cycloalkenyl, von denen jeder Rest gegebenenfalls substituiert sein kann, und $R^{3a}$ ist Wasserstoff, C1-3-Alkyl, und C2-3-Alkenyl oder Alkinyl, gegebenenfalls substituiert durch Cyan, C1-4-Alkylthio, C1-4-Alkoxy oder Halogen.

8. 1-(4-Bromophenyl)-4-cyclohexyl-3-methyl-2,6,7-trioxabicyclo[2,2,2]octan,
1-(4-Chlorophenyl)-3-methyl-4-iso-propyl-2,6,7-trioxabicyclo[2,2,2]octan,
1-(4-Bromophenyl)-3-methyl-4-n-propyl-2,6,7-trioxabicyclo[2,2,2]octan,
1-( 4-Chlorophenyl)-3-methyl-4-n-propyl-2,6,7-trioxabicyclo[2,2,2]octan,
1-cyclohexyl-3-methyl-4-iso-propyl-2,6,7-trioxabicyclo[2,2,2]octan,
1-(4-Bromophenyl)-4-t-butyl-3-methyl-2,6,7-trioxabicyclo[2,2,2]octan,
1,4-Dicyclohexyl-3-methyl-2,6,7-trioxabicyclo[2,2,2]octan,
4-t-Butyl-1-(4-chlorphenyl)-3-methyl-2,6,7-trioxabicyclo[2,2,2]octan,
1-(4-Ethynylphenyl)-3-methyl-4-n-propyl-2,6,7-trioxabicyclo[2,2,2]octan,
3-Methyl-4-n-propyl-1-[4-(2-trimethylsilylethinyl)-phenyl]-2,6,7-trioxabicyclo[2,2,2]octan,
1-Cyclohexyl-3-methyl-4-n-propyl-2,6,7-trioxabicyclo[2,2,2]octan,
4-t-Butyl-1-cyclohexyl-3-methyl-2,6,7-trioxabicyclo[2,2,2]octan,
1-(4-Bromophenyl)-4-n-butyl-3-methyl-2,6,7-trioxabicyclo[2,2,2]octan,
4-n-Butyl-1-(4-chlorophenyl)-3-methyl-2,6,7-trioxabicyclo[2,2,2]octan,
4-n-Butyl-1-(4-indophenyl)-3-methyl-2,6,7-trioxabicyclo[2,2,2]octan,
4-n-Butyl-3-methyl-1-[4-(2-trimethylsilylethinyl)-phenyl]-2,6,7-trioxabicyclo[2.2.2]octane, oder
4-n-Butyl-1-(4-ethinylphenyl)-3-methyl-2,6,7-trioxabicyclo[2,2,2]octan,

**9.** 1-(4-Ethinylphenyl)-4-n-propyl-3-trifluormethyl-2,6,7-trioxabicyclo[2,2,2]octan,
4-n-Propyl-3-trifluormethyl-1-[4-(2-trimethylsilylethinyl)-phenyl-2,6,7-trioxabicyclo[2,2,2]octan,
1-(4-Chlorophenyl)-4-n-propyl-3-trifluormethyl-2,6,7-trioxabicyclo[2,2,2]octan,
1-(4-Bromophenyl)-4-n-propyl-3-trifluormethyl-2,6,7-trioxabicyclo[2,2,2]octan,
1-(4-Chlorophenyl)-4-cyclohexyl-3-trifluormethyl-2,6,7-trioxabicyclo[2,2,2]octan,
1-(4-Bromophenyl)-4-cyclohexyl-3-trifluormethyl-2,6,7-trioxabicyclo[2,2,2]octan,
1-Cyclohexyl-4-n-propyl-3-trifluormethyl-2,6,7-trioxabicyclo[2,2,2]octan,
1-(4-Cyanophenyl)-4-n-propyl-3-trifluormethyl-2,6,7-trioxabicyclo[2,2,2]octan,
1-(4-Bromo-3,5-dichlorphenyl)-4-n-propyl-3-trifluormethyl-2,6,7-trioxabicyclo[2,2,2]octan,
1-(4-Brom-3-chlorophenyl)-4-n-propyl-3-trifluormethyl-2,6,7-trioxabicyclo[2,2,2]octan, oder
4-n-Butyl-1-(4-bromphenyl)-3-trifluormethyl-2,6,7-trioxabicyclo[2,2,2]octan.

**10.** 1-(4-Bromophenyl)-3-ethenyl-4-n-propyl-2,6,7-trioxabicyclo[2,2,2]octan, oder 1-(4-Cyanophenyl)-3-ethinyl-4-isopropyl-2,6,7-trioxabicyclo[2,2,2]octan.

**11.** 1-(4-Bromophenyl)-3-cyano-4-n-propyl-2,6,7-trioxabicyclo[2,2,2]octan,
3-Cyano-1-(4-ethynylphenyl)-4-n-propyl-2,6,7-trioxabicyclo[2,2,2]octan,
4-t-Butyl-3-cyano-1-(4-ethinylphenyl)-2,6,7-trioxabicyclo[2,2,2]octan,
3-Cyano-1-(4-cyanophenyl)-4-isopropyl-2,6,7-trioxabicyclo[2,2,2]octan,
4-t-Butyl-3-cyano-1-(4-iodophenyl)-2,6,7-trioxabicyclo[2,2,2]octan,
4-t-Butyl-3-cyano-1-[4-(2-trimethylsilylethinyl)-phenyl]-2,6,7-trioxabicyclo[2,2,2]octan,
4-t-Butyl-3-cyano-1-cyclohexyl-2,6,7-trioxabicyclo[2,2,2]octan,
1-(4-Bromophenyl)-3-cyano-4-n-propyl-2,6,7-trioxabicyclo[2,2,2]octan,
3-Cyano-1-(4-iodophenyl)-4-n-propyl-2,6,7-trioxabicyclo[2,2,2]octan,
3-Cyano-4-n-propyl-1-[4-(2-trimethylsilylethinyl)phenyl]-2,6,7-trioxabicyclo[2,2,2]octan,
3-Cyano-1-(4-ethinylphenyl)-4-n-propyl-2,6,7-trioxabicyclo[2,2,2]octan.

**12.** 1-(4-Bromophenyl)-4-cyclohexyl-3-methoxymethyl-2,6,7-trioxabicyclo[2,2,2]octan

**13.** 1-(4-Bromophenyl)-4-t-butyl-3-ethyl-2,6,7-trioxabicyclo[2,2,2]octan

**14.** 1-(4-Chlorophenyl)-3,5-dimethyl-4-n-propyl-2,6,7-trioxabicyclo[2,2,2]octan,
1-(4-Bromophenyl)-3,5-dimethyl-4-n-propyl-2,6,7-trioxabicyclo[2,2,2]octan,
1-Cyclohexyl-3,5-dimethyl-4-n-propyl-2,6,7-trioxabicyclo[2,2,2]octan

**15.** Verfahren zur Herstellung einer Verbindung der Formel (I) nach einem der Ansprüche 1 bis 14, dadurch gekennzeichnet, daS es die Kondensation eines Orthocarboxylats der Formel $R^2C(OR^7)_3$ mit einem Triol der Formel (II) umfaßt

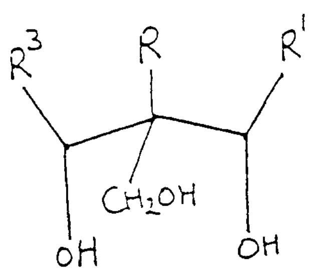

II

worin R bis R3 die in den vorstehenden Ansprüchen angegebene Bedeutung besitzen und R7 C1-4-Alkyl, Phenyl oder C7-8-Aralkyl ist.

**16.** Insectizide oder acarizide Zusammensetzung, umfassend eine Verbindung der Formel (I) nach einem der Ansprüche 1 bis 14 in Mischung mit einem Träger oder Verdünner.

**17.** Synergistische pestizide Zusammensetzung umfassend eine Verbindung der Formel (I) nach einem der Ansprüche 1 bis 14, eine für die Verbindung der Formel (I) synergistisch wirkende Substanz, und einen Träger oder Verdünner.

**18.** Mischung einer Verbindung der Formel (I) nach einem der Ansprüche 1 bis 14 und einer anderen pestiziden Verbindung.

**19.** Verfahren zur Schädlingsbekämpfung, dadurch gekennzeichnet, daß man auf die Schädlinge oder eine gegenüber schädlingsbefall anfällige Umgebung eine Verbindung nach einem der Ansprüche 1 bis 14 oder eine Zusammensetzung oder Mischung nach einem der Ansprüche 16 bis 18 appliziert.

**20.** Verbindung nach einem der Ansprüche 1 bis 14 oder Zusammensetzung nach einem der Ansprüche 16 bis 18 zur Verwendung in einem am menschlichen oder tierischen Körper durchgeführten chirurgischen oder Therapieverfahren oder in einem am menschlichen oder tierischen Körper durchgeführten Diagnoseverfahren.

**21.** Verbindung der Formel

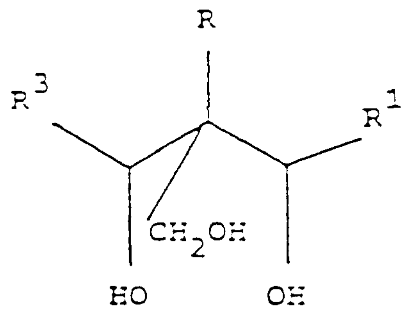

II

worin R, R1 und R3 die in einem der Ansprüche 1 bis 3 oder 6 bis 14 angegebene Bedeutung besitzen, mit der Maßnahme, daß, wenn R1 CH3 und R3 H ist, dann R nicht Ethyl, Isopropyl oder n-Propyl bedeutet.

**22.** Verbindung nach Anspruch 21, dadurch gekennzeichnet, daß, wenn R1 CH3 und R3 H ist, R keine C1-C3-Alkylgruppe bedeutet.

**23.** Verbindung nach Anspruch 21, dadurch gekennzeichnet, daß R CF3 ist.

**Revendications**

**1.** Composé de formule (I) :

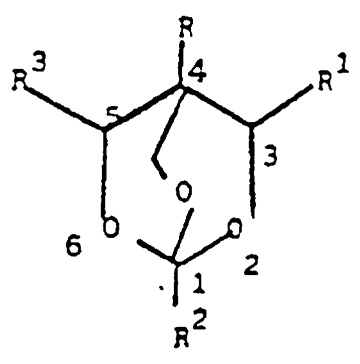

(I)

dans laquelle R est un groupe alkyle, alcényle ou alcynyle en $C_2$-$C_{10}$, chacun étant éventuellement substitué, ou méthyl-substitué, par un groupe cyano, cycloalkyle en $C_3$-$C_4$, halogéno, alcoxy en $C_1$-$C_4$ ou par un groupe $S(O)m^{R4}$, dans lequel $R^4$ est un groupe alkyle en $C_1$-$C_4$ et m est égal à 0 1 ou 2, ou R est un groupe cycloalkyle en $C_3$-$C_{10}$, cycloalcényle en $C_4$-$C_{10}$ ou phényle, chacun étant éventuellement substitué par un groupe alcoxy en $C_1$-$C_4$, alkyle en $C_1$-$C_3$, alcynyle en $C_2$-$C_4$, halogéno, cyano ou par un groupe $S(O)m^{R4}$, tel que défini ci-dessus; $R^1$ est un groupe halogéno, alkyle en $C_1$-$C_3$, alcényle

ou alcynyle en $C_2$-$C_3$, chacun étant éventuellement substitué par un groupe halogéno, cyano, alcoxy en $C_1$-$C_4$, alkylcarbalcoxy contenant jusqu'à 6 atomes de carbone, un groupe $S(O)m^{R4}$, tel que défini ci-dessus, ou alcynyle substitué par un groupe tri(alkyl en $C_1$-$C_4$)silyle, ou $R^1$ est un groupe cyano, spiro-cyclopropyle, gem-diméthyle, gem-dicyano, gem-diéthynyle, oxo ou méthylène, éventuellement substitué par un groupe cyano ou alkyle en $C_1$-$C_3$ éventuellement substitué par un fluor, ou bien $R^1$ et R, et les atomes de carbone auxquels ils sont fixés, forment un noyau carbocyclique en $C_5$-$C_7$, éventuellement substitué par un groupe halogéno, alkyle ou alcoxy en $C_1$-$C_3$ ou alcényle en $C_2$-$C_3$, $R^2$ est un groupe phényle, cycloalkyle ou cycloalcényle en $C_5$-$C_{10}$, chacun étant éventuellement substitué par un groupe halogéno, cyano, azido, tétrazolyle, un groupe $SO_2R^5$, dans lequel $R^5$ est un groupe amino ou di(alkylamino en $C_1$-$C_4$), un groupe $COR^6$, dans lequel $R^6$ est un groupe alcoxy en $C_1$-$C_4$, benzyloxy, amino ou di(alkylamino en $C_1$-$C_4$), nitro, alkyle en $C_1$-$C_3$, éventuellement substitué par un groupe halogéno, cyano ou éthynyle, ou alcényle en $C_2$-$C_3$ ou éthynyle, chacun étant éventuellement substitué par un groupe halogéno ou tri(alkyl en $C_1$-$C_4$)silyle, et $R^3$ est un hydrogène, un groupe alkyle en $C_1$-$C_3$, alcényle ou alcynyle en $C_2$-$C_3$, chacun étant éventuellement substitué par un groupe cyano, alkylthio en $C_1$-$C_4$, alcoxy en $C_1$-$C_4$ ou halogéno, ou $R^3$ est un groupe cyano ou halogéno.

**2.** Composé selon la revendication 1, dans lequel R est un groupe n-propyle, n-butyle, iso-butyle, s-butyle, t-butyle, cyclopentyle ou cyclohexyle.

**3.** Composé selon l'une quelconque des revendications 1 ou 2, dans lequel $R^1$ est un groupe méthyle, éthyle, trifluorométhyle, cyano ou éthynyle.

**4.** Composé selon l'une quelconque des revendications 1 à 3, dans lequel $R^2$ est un groupe cyclohexyle, cycloheptyle, cyclooctyle ou phényle, éventuellement substitué en positions 3, 4 et/ou 5 par un groupe halogéno, cyano, éthynyle, azido ou nitro et/ou en positions 2 et/ou 6 par un fluor.

**5.** Composé selon la revendication 4, dans lequel $R^2$ est un groupe phényle substitué en position 4 par un chlore, un brome ou un groupe éthynyle.

**6.** Composé selon l'une quelconque des revendications 1 à 5, dans lequel $R^3$ est un hydrogène ou un groupe méthyle.

**7.** Composé de formule IA :

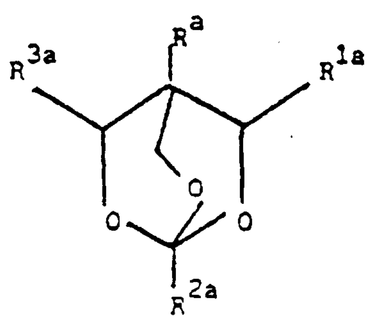

(IA)

dans laquelle $R^a$ est un groupe alkyle, alcényle ou alcynyle en $C_2$-$C_4$, cycloalkyle en $C_5$-$C_{10}$ ou phényle, chacun étant éventuellement substitué par un groupe cyano ou alcoxy en $C_1$-$C_4$, $R^{1a}$ est un groupe cyano ou alkyle en $C_1$-$C_3$, alcényle ou alcynyle en $C_2$-$C_3$, chacun étant éventuellement substitué par un groupe cyano, alcoxy en $C_1$-$C_4$, alkylthio en $C_1$-$C_4$ ou halogéno, ou $R^{1a}$ est un groupe cyano, gem-diméthyle, ou $R^{1a}$ et $R^a$, et les atomes de carbone auxquels ils sont fixés, forment un noyau carbocyclique en $C_5$-$C_7$, éventuellement substitué par un groupe alkyle ou alcoxy en $C_1$-$C_3$; $R^{2a}$ est un groupe phényle, cycloalkyle ou cycloalcényle en $C_5$-$C_{10}$,chacun étant éventuellement substitué, et $R^{3a}$ est un hydrogène, un groupe alkyle en $C_1$-$C_3$, alcényle ou alcynyle en $C_2$-$C_3$, chacun étant éventuellement substitué par un groupe cyano, alkylthio en $C_1$-$C_4$, alcoxy en $C_1$-$C_4$ ou halogéno.

**8.** Le 1-(4-bromophényl)-4-cyclohexyl-3-méthyl-2,6,7-trioxabicyclo[2,2,2]octane,
le 1-(4-chlorophényl)-3-méthyl-4-isopropyl-2,6,7-trioxabicyclo[2,2,2]octane,

le 1-(4-bromophenyl)-3-méthyl-4-n-propyl-2,6,7-trioxabicyclo[2,2,2]octane,
le 1-(4-chlorophényl)-3-méthyl-4-n-propyl-2,6,7-trioxabicyclo[2,2,2]octane,
le 1-cyclohexyl-3-méthyl-4-isopropyl-2,6,7-trioxabicyclo[2,2,2]octane,
le 1-(4-bromophényl)-4-t-butyl-3-méthyl-2,6,7-trioxabicyclo[2,2,2]octane,
le 1,4-dicyclohexyl-3-méthyl-2,6,7-trioxabicyclo[2,2,2]-octane,
le 4-t-butyl-1-(4-chlorophényl)-3-méthyl-2,6,7-trioxabicyclo[2,2,2]octane,
le 1-(4-éthynylphényl)-3-méthyl-4-n-propyl-2,6,7-trioxabicyclo[2,2,2]octane,
le 3-méthyl-4-n-propyl-1-[4-(2-triméthylsilyléthynyl)-phényl]-2,6,7-trioxabicyclo[2,2,2]octane,
le 1-cyclohexyl-3-méthyl-4-n-propyl-2,6,7-trioxabicyclo[2,2,2]octane,
le 4-t-butyl-1-cyclohexyl-3-méthyl-2,6,7-trioxabicyclo[2,2,2]octane,
le 1-(4-bromophényl)-4-n-butyl-3-méthyl-2,6,7-trioxabicyclo[2,2,2]octane,
le 4-n-butyl-1-(4-chlorophényl)-3-méthyl-2,6,7-trioxabicyclo[2,2,2]octane,
le 4-n-butyl-1-(4-indophényl)-3-méthyl-2,6,7-trioxabicyclo[2,2,2]octane,
le 4-n-butyl-3-méthyl-1-[4-(2-triméthylsilyléthynyl)-phényl]-2,6,7-trioxabicyclo[2,2,2]octane, ou
le 4-n-butyl-1-(4-éthynylphényl)-3-méthyl-2,6,7-trioxabicyclo[2,2,2]octane,

**9.** Le 1-(4-éthynylphényl)-4-n-propyl-3-trifluorométhyl-2,6,7-trioxabicyclo[2,2,2]octane,
le 4-n-propyl-3-trifluorométhyl-1-[4-(2-triméthylsilyléthynyl)phényl]-2,6,7-trioxabicyclo[2,2,2]octane,
le 1-(4-chlorophényl)-4-n-propyl-3-trifluorométhyl-2,6,7-trioxabicyclo[2,2,2]octane,
le 1-(4-bromophényl)-4-n-propyl-3-trifluorométhyl-2,6,7-trioxabicyclo[2,2,2]octane,
le 1-(4-chlorophényl)-4-cyclohexyl-3-trifluorométhyl-2,6,7-trioxabicyclo[2,2,2]octane,
le 1-(4-bromophényl)-4-cyclohexyl-3-trifluorométhyl-2,6,7-trioxabicyclo[2,2,2]octane,
le 1-cyclohexyl-4-n-propyl-3-trifluorométhyl-2,6,7-trioxabicyclo[2,2,2]octane,
le 1-(4-cyanophényl)-4-n-propyl-3-trifluorométhyl-2,6,7-trioxabicyclo[2,2,2]octane,
le 1-(4-bromo-3,5-dichlorophényl)-4-n-propyl-3-trifluorométhyl-2,6,7-trioxabicyclo[2,2,2]octane,
le 1-(4-bromo-3-chlorophényl)-4-n-propyl-3-trifluorométhyl-2,6,7-trioxabicyclo[2,2,2]octane, ou
le 4-n-butyl-1-(4-bromophényl)-3-trifluorométhyl-2,6,7-trioxabicyclo[2,2,2]octane.

**10.** Le 1-(4-bromophényl)-3-éthényl-4-n-propyl-2,6,7-trioxabicyclo[2,2,2]octane, ou
le 1-(4-cyanophényl)-3-éthynyl-4-isopropyl-2,6,7-trioxabicyclo[2,2,2]octane.

**11.** Le 1-(4-bromophényl)-3-cyano-4-n-propyl-2,6,7-trioxabicyclo[2,2,2]octane,
le 3-cyano-1-(4-éthynylphényl)-4-n-propyl-2,6,7-trioxabicyclo[2,2,2]octane,
le 4-t-butyl-3-cyano-1-(4-éthynylphényl)-2,6,7-trioxabicyclo[2,2,2]octane,
le 3-cyano-1-(4-cyanophényl)-4-isopropy1-2,6,7-trioxabicyclo[2,2,2]octane,
le 4-t-butyl-3-cyano-1-(4-iodophényl)-2,6,7-trioxabicyclo[2,2,2]octane,
le 4-t-butyl-3-cyano-1-[4-(2-triméthylsilyléthynyl)-phényl]-2,6,7-trioxabicyclo[2,2,2]octane,
le 4-t-butyl-3-cyano-1-cyclohexyl-2,6,7-trioxabicyclo[2,2,2]octane,
le 1-(4-bromophényl)-3-cyano-4-n-propyl-2,6,7-trioxabicyclo[2,2,2]octane,
le 3-cyano-1-(4-Iodophényl)-4-n-propyl-2,6,7-trioxabicyclo[2,2,2]octane,
le 3-cyano-4-n-propyl-1-[4-(2-triméthylsilyléthynyl)--phényl]-2,6,7-trioxabicyclo[2,2,2]octane, ou
le 3-cyano-1-(4-éthynylphényl)-4-n-propyl-2,6,7-trioxabicyclo[2,2,2]octane.

**12.** Le 1-(4-bromophényl)-4-cyclohexyl-3-méthoxyméthyl-2,6,7-trioxabicyclo[2,2,2]octane.

**13.** Le 1-(4-bromophényl)-4-t-butyl-3-éthyl-2,6,7-trioxabicyclo[2,2,2]octane.

**14.** Le 1-(4-chlorophényl)-3,5-diméthyl-4-n-propyl-2,6,7-trioxabicyclo[2,2,2]octane,
le 1-(4-bromophényl)-3,5-diméthyl-4-n-propyl-2,6,7-trioxabicyclo[2,2,2]octane, ou
le 1-cyclohexyl-3,5-diméthyl-4-n-propyl-2,6,7-trioxabicyclo[2,2,2]octane.

**15.** Procédé de préparation d'un composé de formule (I) telle que définie dans l'une quelconque des revendications 1 à 14, qui comprend la condensation d'un orthocarboxylate de formule $R^2C(OR^7)_3$ avec un triol de formule (II) :

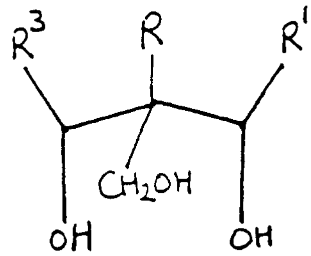

II

dans laquelle R à $R^3$ sont tels que définis ci-dessus et $R^7$ est un groupe alkyle en $C_1$-$C_4$, phényle ou arylalkyle en $C_7$-$C_8$.

**16.** Composition insecticide ou acaricide comprenant un composé de formule (I) telle que définie dans l'une quelconque des revendications 1 à 14 en mélange avec un véhicule ou un diluant.

**17.** Composition pesticide synergique comprenant un composé de formule (I) telle que définie dans l'une quelconque des revendications 1 à 14, un agent synergique pour le composé de formule I et un véhicule ou un diluant.

**18.** Mélange d'un composé de formule (I) telle que définie dans l'une quelconque des revendications 1 à 14 et d'un autre composé pesticide.

**19.** Procédé de lutte contre les nuisibles, comprenant l'application sur les nuisibles, ou sur un environnement susceptible d'être infesté par des nuisibles, d'un composé selon l'une quelconque des revendications 1 à 14 ou d'une composition ou d'un mélange selon l'une quelconque des revendications 16 à 18.

**20.** Composé tel que défini dans l'une quelconque des revendications 1 à 14 ou composition selon l'une quelconque des revendications 16 à 18 à utiliser dans un procédé de chirurgie ou de thérapeutique pratiqué sur un organisme humain ou animal, ou dans un procédé de diagnostic pratiqué sur un organisme humain ou animal.

**21.** Composé de formule :

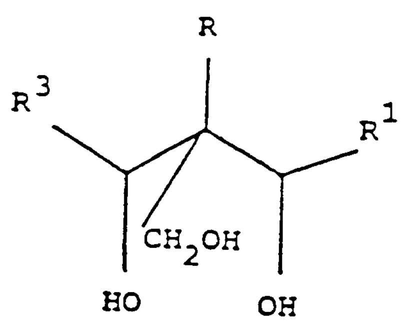

II

dans laquelle R, $R^1$ et $R^3$ sont tels que définis dans l'une quelconque des revendications 1-3, ou 6 à 14, à la condition que, lorsque $R^1$ est $CH_3$ et que $R^3$ est H, R ne soit pas un groupe éthyle, isopropyle ou n-propyle.

**22.** Composé selon la revendication 21, dans lequel, lorsque $R^1$ est $CH_3$ et que $R^3$ est H, R n'est pas un groupe alkyle en $C_1$-$C_3$.

**23.** Composé selon la revendication 21, dans lequel R est $CF_3$.